(19) Europäisches Patentamt
European Patent Office
Office européen des brevets

(11) Publication number: 0 270 234 A2

(12) EUROPEAN PATENT APPLICATION

(21) Application number: 87309462.7

(22) Date of filing: 27.10.87

(51) Int. Cl.⁴: C07K 5/02 , A61K 37/64 , C07D 405/14 , A61K 31/395 , A61K 31/335 , //(C07D405/14,307:00,307:00,233:00),(C07D405/14,319:00,307:00,233:00),(C07D405/14,311:00,307:00,233:00),(C07D405/14,307:00,307:00,233:00,207:00),(C07D405/14,319:00,307:00)

The title of the invention has been amended (Guidelines for Examination in the EPO, A-III, 7.3).

(30) Priority: 31.10.86 PCT/US86/02348

(43) Date of publication of application: 08.06.88 Bulletin 88/23

(84) Designated Contracting States: AT BE CH DE ES FR GB GR IT LI LU NL SE

(71) Applicant: PFIZER INC.
235 East 42nd Street
New York, N.Y. 10017(US)

(72) Inventor: Rosati, Robert Louis
RFD 1, Box 66A Dean's Mill Road
Stonington Connecticut(US)

(74) Representative: Graham, Philip Colin Christison et al
Patents Department Pfizer Limited Ramsgate Road
Sandwich, Kent CT13 9NJ(GB)

(54) **Renin inhibitors.**

(57) Polypeptides of relatively low molecular weight and small size; namely derivatives of 4-amino-3-hydroxy-4-substituted butanoic acids and 5-amino-2,5-disubstituted-4-hydroxypentunoic acids for use as inhibitors of the cleavage of angiotensinogen by renin and intermediates therefor.

EP 0 270 234 A2

Xerox Copy Centre

87309462.7

PC(Ph)7097

# RELATIVELY LOW MOLECULAR WEIGHT POLYPEPTIDES

This invention relates to novel polypeptides of relatively low molecular weight and size; namely, derivatives of 4-amino-3-hydroxy-4-substituted butanoic acids or of 5-amino-2,5-disubstituted-4-hydroxypentanoic acids, as renin inhibitors. More particularly, it relates to said polypeptides, to certain intermediates therefor, and the use of the polypeptides to inhibit the angiotensinogen-cleaving action of the enzyme renin.

The proteolytic enzyme renin (EC 3.4.23.15), which has a molecular weight of about 40,000, is produced in and secreted into the blood by the kidney. It is known to be active in vivo in selectively cleaving the naturally-occurring plasma glycoprotein angiotensinogen. In the case of human angiotensinogen, cleavage is at the bond between the leucine (10th) and valine (11th) amino acid residues at the N-terminal end of the angiotensinogen:

Asp-Arg-Val-Tyr-Ile-His-Pro-Phe-His-Leu-Val-
1 2 3 4 5 6 7 8 9 10 11
Ile-His-Ser-Glu-
12 13 14 15

The circulating N-terminal decapeptide (angiotensin I) formed by the above cleaving action of renin is subsequently converted by the body to an octapeptide known as angiotensin II. Angiotensin II is a potent pressor substance, i.e., a substance that is capable of inducing a significant increase in blood pressure, and is believed to act by causing the constriction of blood vessels and the release of the sodium-retaining hormone aldosterone from the adrenal gland. Thus, the

renin-angiotensinogen system has been implicated as a causative factor in certain forms of hypertension.

One means of alleviating the adverse effects of the functioning of the renin-angiotensinogen system is the administration of a substance capable of inhibiting the angiotensinogen-cleaving action of renin. A number of such substances are known, including anti-renin antibodies, pepstatin and naturally-occurring phospholipid compounds. U.S. Patent 4,478,826, issued October 23, 1984, discloses a series of renin-inhibiting polypeptide compounds having a non-terminal statine (Sta; 4-amino-3-hydroxy-6-methylheptanoic acid) or statine derivative. Including Sta, the vast majority of compounds exemplified contain 6 or more amino acid residues. Exemplary of the few shortest chains there disclosed are:

Acetyl-Phe-His-Sta-Leu-Phe-$NH_2$, and

t-Butyloxycarbonyl-Phe-His-Sta-Leu-Phe-$NH_2$.

There are invariably at least two amino acid residues each side of statine. The di- or polypeptidyl-statyl group is invariably attached to a lipophilic amino acid, most often leucine. (See also U.S. Patents 4,470,971 and 4,478,826).

European Patent Application No. 45,665 and U.S. Patent 4,424,207 disclose a series of renin-inhibiting polypeptide derivatives of the formula

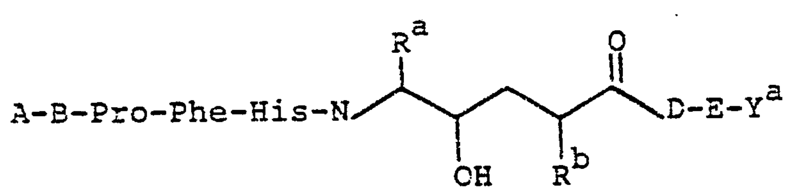

where A is for example t-butoxycarbonyl, B is His or other basic aminoacyl group, D is Val, Ile or other

liphophilic aminoacyl residue, E is Tyr, Phe, His or other aromatic aminoacyl residue, $R^a$ and $R^b$ are each isopropyl, isobutyl, benzyl or other lipophilic amino-acid type sidechain, and $Y^a$ is a terminal acid, ester or amide type group. Including the central 5-aminopentanoic acid residues, these compounds are invariably heptapeptides, i.e., N-tetrapeptidyl-5-aminopentanoyllipophilic aminoacyl-aromatic amino-acid derivatives.

European Patent Application 132,304A also discloses the use of statine containing polypeptides as renin-inhibiting antihypertensive agents, and European Patent Application 114,993A discloses polypeptides containing cyclostatine, useful as renin-inhibiting antihypertensive agents.

It has now been found that certain peptides of relatively small size and relatively low molecular weight are valuable renin-inhibiting agents. The compounds have the formula (I):

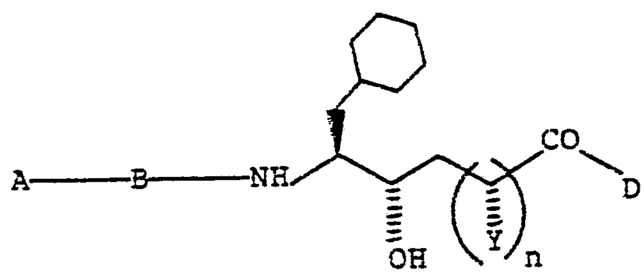

wherein A is

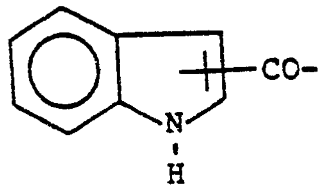 ,

(A-1)

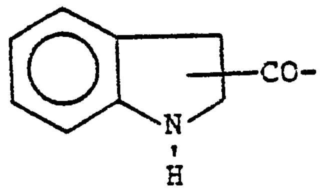 ,

(A-2)

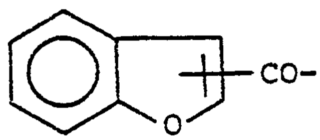 ,

(A-3)

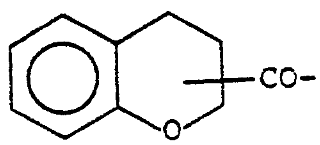 ,

(A-4)

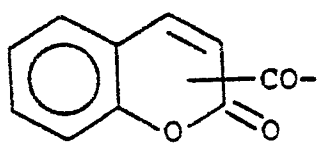 ,

(A-5)

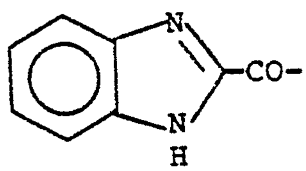 ,

(A-6)

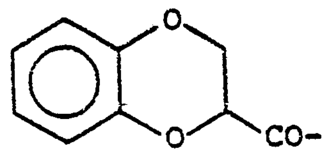 ,

(A-7)

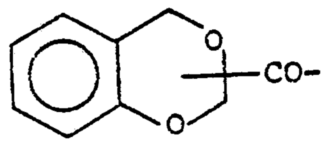 or

(A-8)

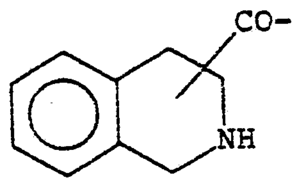 ;

(A-9)

n is 0 or 1;

B is an aromatic or aliphatic amino acid moiety which is attached to group A and the -NH group of the remaining segment of (I) through its amino and carboxyl groups respectively, and is derived from (i) alanine, (ii) norleucine, or (iii) histidine; said moieties having the formulae:

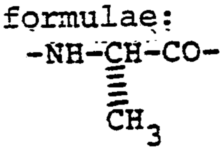 ,

(B-1)

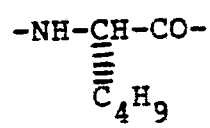 and

(B-2)

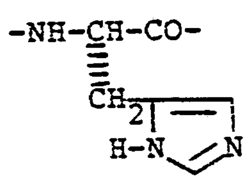 ;

(B-3)

Y is isobutyl, allyl or benzyl;

D is

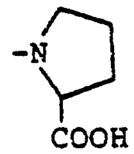 or -Z-R

wherein -Z- is -O- or -NH-; and

R is $(C_{1-6})$alkyl or phenyl each of which can be optionally substituted by amino, hydroxyl, carboxyl or carbo$(C_{1-6})$alkoxy provided that when said substituent is amino or hydroxyl, R is $(C_{2-6})$alkyl.

The internal moiety of formula (I) which is represented structurally in (I), includes the following:

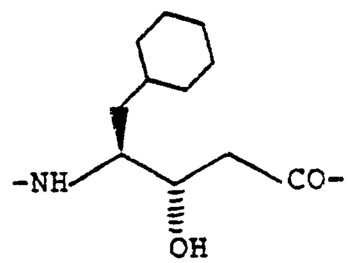

(a)

and

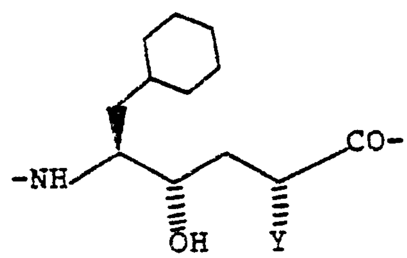

.

(b)

Amino acid residue (a), above, is derived from 3S,4S-cyclohexyl statine and (b) from 4S,5S-homocyclohexyl statine, the 2-position of which is substituted by isobutyl (2-methylpropyl), allyl or benzyl. They and other amino acid residues or groups will, for the sake of brevity and convenience, be designated herein by the commonly accepted abbreviations listed below:

| Abbreviation | Amino Acid |
|---|---|
| cSta | 3S,4S-cyclohexylstatine |
| hcSta | 4S,5S-homocyclohexylstatine |
| ahcSta | 4S,5S-allylhomocyclohexylstatine |
| bhcSta | 4S,5S-benzylhomocyclohexylstatine |
| ibhcSta | 4S,5S-isobutylhomocyclohexylstatine |
| His | L-histidine |
| Ala | L-alanine |
| Phe | L-phenylalanine |
| Nle | L-norleucine |
| GABA | 4-aminobutanoic acid |
| BOC | t-butoxycarbonyl |
| CBz | carbobenzoxy |
| Bz | benzyl |

Also included in this invention are the pharmaceutically acceptable salts of formula (I) compounds. By the term "pharmaceutically acceptable" salts is meant acid addition salts of any of the compounds of the formula (I) which contain a basic functionality with an acid such as (but not limited to) HCl, $HNO_3$, $H_2SO_4$, $H_3PO_4$, $CH_3SO_3H$, p-$CH_3C_6H_4SO_3H$, $CH_3COOH$ or $HOOCCH_2CH_2COOH$. When the compound (I) contains more than one basic function, the salt optionally contains more than one equivalent of acid. Alternatively, when the compound of the formula I contains an acidic function, the expression also refers to cationic salts such as, but not limited to, an alkali metal salt (e.g., Na, K), an alkaline earth salt (e.g., Ca, Mg) or an amine salt (e.g., diethanolamine, meglumine). Conventional methods are used to prepare such salts.

The present invention also encompasses pharmaceutical compositions containing a renin inhibiting effective amount of a compound of the formula (I) as the essential active ingredient in a pharmaceutically acceptable carrier; and a method for inhibiting the cleavage of angiotensinogen by renin in the body of a mammal which comprises administering to the mammal a renin inhibiting effective amount of a compound of the formula (I).

Additionally, the present invention includes intermediate compounds of the formula (II) and (III)

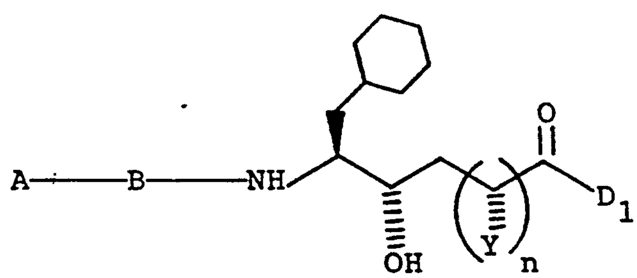

(II)

and

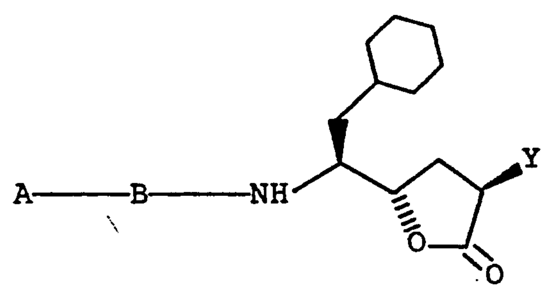

(III)

wherein each of A, B, Y and n is as defined above; and

$D_1$ is $(C_{1-6})$alkoxy, benzyloxy or hydroxy;

and processes for making compounds of formulae (I), (II) and (III).

Favored values of the variables of formula (I) compounds are the following:

A: (A-1), (A-4), (A-5), (A-6) and (A-7);

Y: isobutyl and allyl;

n: 0 and 1.

Preferred values of formula (I) compounds are those favored compounds wherein:

A: (A-1), (A-6) and (A-7);

B: (B-2) and (B-3);

D: ZR wherein Z is -NH, and R is $(C_{1-6})$alkyl optionally substituted;

Y: isobutyl;

n: 1.

Especially preferred compounds of formula (I) are those preferred compounds having the values:

A is (A-1) and (A-6);

B is (B-3); and

D is ZR wherein R is $-CH_3$.

The compounds of this invention are prepared by methods familiar to those skilled in the art. These general methods are illustrated in the reaction sequence below wherein, for simplicity, formula (I) compounds are represented by the formula

$$A—B—C—D \quad (I\text{-}A)$$

wherein A, B and D are as previously defined and C represents the unit

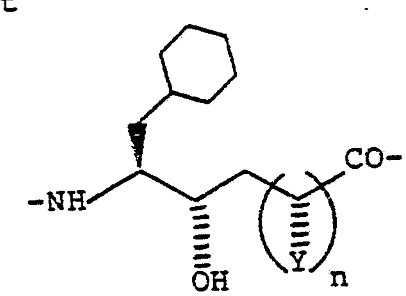

wherein n and Y are as defined above.

As the skilled artisan will recognize, formula (I-A) compounds can be assembled in a variety of ways. For example, the individual units can be joined by a series of stepwise reactions such as A + B to produce A-B which is then reacted with unit C and the resulting product A-B-C then reacted with D. Alternatively, the reaction sequence can begin by combining D and C followed by successive reactions with B and A. In a further modification A-B can be combined with C-D, or with C followed by reaction with D. Still further, B-C can be reacted with A or D and the resulting product combined with D or A respectively.

The general methodology involves formation of peptide (or amide, -CONH-) linkages between amino and carboxylic acid groups. Units B and C of formula (I) are derived from amino acids; unit A from a carboxylic acid and unit D from an amine. Of course, when D is ZR wherein Z is -O-, the reaction linking C with D is an esterification reaction.

While a variety of methods can be employed to form the peptide linkages, a favored method comprises dehydrative coupling between the appropriate reactants as described below.

In the examples presented herein, certain protecting and activating groups are specifically illustrated. However, one skilled in the art will recognize that other protecting or activating groups could have been used. The choice of a particular protecting group is dependent to a great extent upon the availability of the necessary reagent, its effect upon solubility of the "protected" compound, its ease of removal and the presence of other groups which might be affected by its use; i.e., its selectivity, or its removal.

For example, it will be necessary, or at least desirable, in many reactions to protect the amino groups and/or the carboxy groups. The synthetic route chosen for the preparation of formula (I) compounds may require removal of one or the other or both of said protecting groups in order to permit further reaction at the regenerated amino or carboxy group; i.e., the protecting groups used are, in most instances, removable independently of each other. Additionally, the choice of protecting group for a given amino group depends upon the role of said amino group in the overall reaction scheme. Amino protecting groups having varying levels of lability, i.e., ease of removal, will be used. The same is true as regards carboxy protecting groups. Such groups are known in the art and attention is directed to the reviews by Bodansky et al., "Peptide Synthesis", 2nd Ed., John Wiley & Sons, N.Y. (1976); Greene, "Protective Groups in Organic Synthesis", John Wiley & Sons, N.Y. (1981); McOmie, "Protective Groups in Organic Chemistry", Plenum Press, N.Y. (1973); and to Sheppard in "Comprehensive Organic Chemistry, The Synthesis and Reactions of Organic Compounds", Pergaman Press, N.Y. (1979), edited by E. Haslam, Part 23.6, pages 321-339.

Conventional amino and carboxy protecting groups are known to those skilled in the art. Representative amino protecting groups, but by no means limiting thereof, are the following: such as benzyloxycarbonyl; t-butoxycarbonyl; substituted or unsubstituted aralkyl such as benzyl, trityl, benzhydryl and 4-nitrobenzyl; benzylidene; arylthio such as phenylthio, nitrophenylthio and trichlorophenylthio; phosphoryl derivatives such as dimethylphosphoryl and O,O-dibenzylphosphoryl; trialkylsilyl derivatives such as trimethylsilyl; and others as are described in U.S. Patent No. 4,322,341 and which

are incorporated herein by reference. The preferred amino protecting group is t-butoxycarbonyl. Procedures for substituting said group on a given amino group are well known. In general they comprise acylating the appropriate amino compound with a t-butoxycarbonyl acylating agent, especially of the formula

$$(CH_3)_3C-O-\overset{O}{\overset{\|}{C}}-R_1 \quad ,$$

wherein $R_1$ is F, $N_3$ or $O-CO-OC(CH_3)_3$. The reaction conditions for introducing the BOC protecting group are well known in the art. The preferred acylating agent is that wherein $R_1$ is $O-CO-OC(CH_3)_3$.

The value of the BOC group as protecting agent in the processes described herein resides in its relative ease of removal (deblocking) under relatively mild acid conditions.

It is especially valued when B represents the histidine moiety since it is readily removed from the imidazolyl moiety by treatment with potassium carbonate in methanol without removal of other BOC groups which might be present.

Representative carboxy protecting groups which can be used are various esters such as silyl esters, including trialkyl silyl esters, trihalosilyl esters and haloalkylsilyl esters; certain hydrocarbyl esters such as $(C_{1-4})$alkyl, especially t-butyl groups; benzyl and substituted benzyl esters, benzhydryl and trityl; phenacyl and phthalimidomethyl esters; certain substituted hydrocarbyl esters such as chloromethyl, 2,2,2-trichloroethyl, cyanomethyl; tetrahydropyranyl; methoxymethyl; methylthiomethyl; protected carbazoyl such as $-CONH-NHR^2$ wherein $R^2$ is an amino protecting group as disclosed above, especially benzyloxycarbonyl;

and others as are described in U.S. Patent No. 4,322,341 and which are incorporated herein by reference. The preferred carboxy protecting group is $-CONH-NHR^2$ wherein $R^2$ is benzyloxycarbonyl, said preferred group being referred to as benzyloxycarbonylcarbazide. A highly favored carboxy protecting group is the benzyl group.

The protected carboxy groups are converted to the unprotected carboxy groups by procedures known to those skilled in the art. The benzyl group, the preferred protecting group for carboxy groups is removed by catalytic hydrogenation over a noble metal catalyst, especially over palladium hydroxide-on-carbon or palladium-on-carbon.

When B in formula (I) represents His, the preferred method for producing said compounds comprises the following reaction:

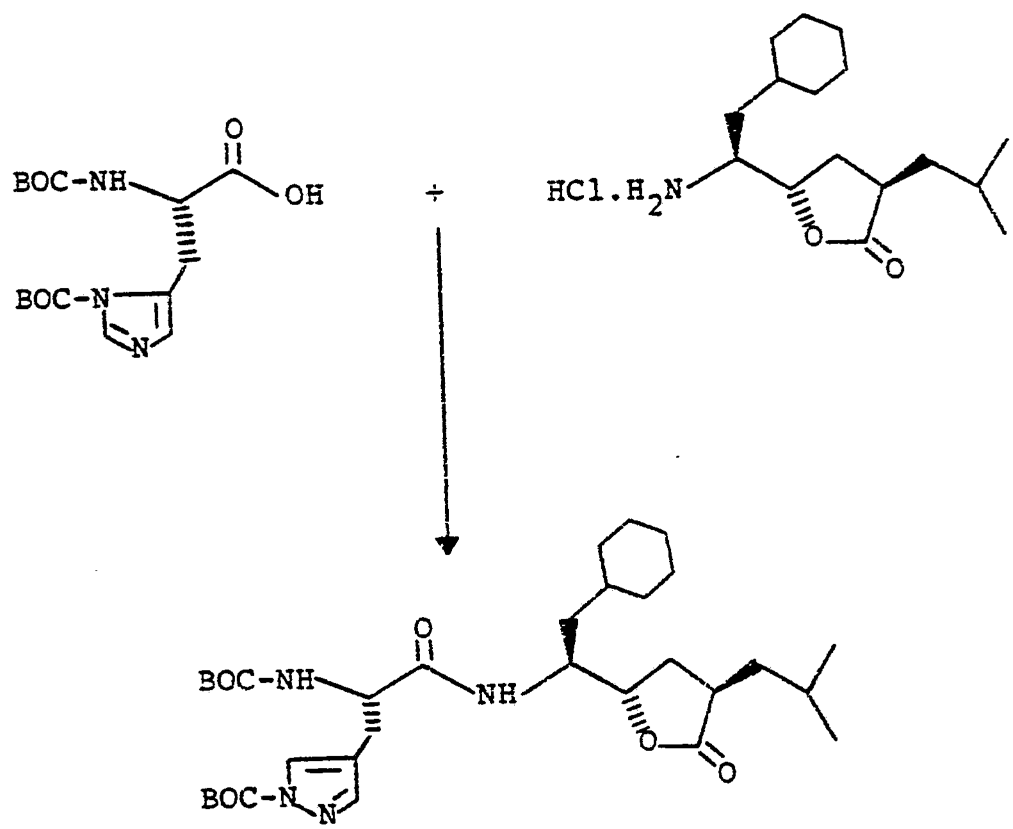

The reactants are combined in equimolar amounts in a reaction-inert solvent in the presence of an acid acceptor such as N-methylmorpholine or other tertiary amine base. A condensing or dehydrative coupling agent, e.g., a carbodiimide such as dicyclohexylcarbodiimide, N,N'-carbonyldiimidazole, N-ethyl-5-phenylisoxazolene-3'-sulfonate, diethylcyanophosphonate and others known in the art, is used to achieve formation of the amide group. In order to minimize racemization during the coupling reaction, 1-hydroxybenzotriazole is added to the reaction mixture.

One skilled in the art will recognize that protecting groups other than BOC can be used on the histidine reactant and that the protecting groups need not be alike.

The lactone containing unit, the precursor to the B-C units of formula (I), is deblocked if necessary, and the deblocked compound reacted with the desired A-COOH reactant to produce a compound of formula (III). Said compound is then reacted with the appropriate amine, amino acid or alcohol to cleave the lactone ring to produce formula (I) compounds. The reaction is conducted in a reaction-inert solvent, i.e., a solvent which does not react adversely with reactants or products in a manner which reduces yield of the desired product. Representative of such solvents are lower alkanols, methylene chloride, dimethylformamide, dioxane or mixtures thereof. The amino group of the amine containing reactant (e.g., amine, amino acid) reacts sufficiently fast with the lactone as to permit the use of alcohols as solvent.

When unit B is other than His, the methodology described for B as His can also be used. Alternatively,

an appropriate C reactant (e.g., BOC cSta, BOC hcSta, BOC ibhcSta) is reacted directly with the appropriate D reactant via peptide bond formation when D is ZR wherein Z is -NH-, or via esterification when Z is -O-.

When it is desired to produce a formula (I) compound wherein Z is -O-, the lactone is reacted with excess alcohol, OH containing reactant, in the presence of a base such as potassium carbonate or a tertiary amine such as triethylamine.

Formula (I) compounds wherein the B moiety is derived from phenylalanine are prepared according to procedures described and exemplified herein for other values of B. Formula (I) compounds wherein Y is benzyl are also made according to procedures disclosed herein. The formula (I) compounds bearing such substituents have the utility disclosed herein and are used in the manner described in this specification.

The activity of the compounds of the present invention as inhibitors of the angiotensinogen-cleaving activity of renin is determined by studying (1) their ability to inhibit the angiotensinogen-cleaving activity of renin *in vitro*. In this test, blood plasma is obtained from healthy laboratory personnel, pooled and stored frozen until required. Before use, a quantity of this plasma is defrosted and centrifuged, and the supernatant mixed with protease inhibitors and buffered to pH 7.4. Renin inhibitors are added at different concentrations to aliquots of the prepared plasma, and the resulting mixtures (310 lambda) incubated for three hours at 37° C. along with renin inhibitor-free control mixtures. After incubation, the mixtures are quenched in ice water and each assayed for angiotensin I using angiotensin I antibody. The angiotensin I in the quenched incubation mixtures is assayed by means of

a radioimmunoassay kit supplied by Becton Dickinson and Co. (Orangeburg, N.Y.). This radioimmunoassay was based upon the one developed by Haber et al., J. Clin. Endocrinol., 29, pp. 1349-1355 (1969). The production of angiotensin I in the presence of a renin inhibitor is compared to that produced in the absence of the inhibitor, and a percentage inhibition is calculated. Using data obtained from duplicate incubations at each of several different inhibitor concentrations, the inhibitor concentration in the incubation mixture required to produce a fifty percent inhibition of the angiotensinogen-cleaving activity of renin, i.e., the $IC_{50}$ of the inhibitor, is calculated for various different inhibitors.

The activity of the present compounds may also be determined by their ability to antagonize the exogenous renin-induced pressor response *in vivo*. Male Sprague-Dawley rats (230 to 300 g body weight) are anesthetized with sodium pentobarbital (65 mg/kg body weight, intraperitoneal), after which femoral vein and carotid artery catheters are implanted in each animal. Following completion of surgery, the animals are placed in the prone position and rectal temperature monitored continuously. Mean arterial blood pressure (MAP) is recorded via the carotid artery catheter using a Statham P23 ID pressure transducer and a physiograph. Subsequent to a stabilization period, control renin pressor responses (dP) of 20 to 30 mm HG are obtained by administration of hog renin (30 to 80 mU/kg body weight, intravenous), the animals are rechallenged with hog renin (same dosage as for control response) at 5, 15 and 30 minutes after renin inhibitor administration

and the corresponding renin pressor responses (dP) measured. Percent antagonization is calculated as

$$\frac{\text{(control dP - experimental dP) x 100\%}}{\text{control dP}},$$

where control dP and experimental dP are the pressor changes in MAP before and after renin inhibitor administration, respectively. Preferably, at least three animals are used in each test, with results averaged.

The compounds of the present invention can be administered as antihypertensive agents by either the oral or parental routes of administration, and are routinely effective by the latter route, particularly when dosed as an intravenous solution. Where gastrointestinal adsorption permits, oral administration is preferred for reasons of patient convenience and comfort. In general, these antihypertensive compounds are normally administered in dosages ranging from about 0.1 mg to about 10 mg per kg of body weight per day; variations will necessarily occur depending upon the condition of the subject being treated and the particular compound being administered. Typically, treatment is commenced at a low daily dosage and increased by the physician only if necessary. It is to be noted that these compounds may be administered in combination with pharmaceutically acceptable carriers by either of the routes previously indicated, and that such administration can be carried out in both single and multiple dosages.

For parenteral use, the present compounds are formulated according to the known art using suitable dispersing or wetting agents and suspending agents. The sterile injectable formulation can also be a solution or suspension in a non-toxic parenterally acceptable diluent or solvent; for example, as a

solution in 1,3-butandiol. Among the acceptable vehicles and solvents are water, Ringer's solution and isotonic NaCl solution, fixed oils including synthetic mono- or diglycerides, fatty acids such as oleic acids, and mixtures thereof.

For oral administration, a wide variety of dosage forms are used, e.g., tablets, capsules, lozenges, troches, hard candies, powders, sprays, aqueous suspensions, elixirs, syrups, and the like formulated with various pharmaceutically-acceptable inert carriers. Such carriers include solid diluents or fillers, sterile aqueous media and various non-toxic organic solvents, etc. In general, the compounds of this invention are present in such oral dosage forms at concentration levels ranging from about 0.5% to about 90% by weight of the total composition, in amounts which are sufficient to provide the desired unit dosage. Tablets may contain various excipients such as sodium citrate, calcium carbonate and calcium phosphate, along with various disintegrants such as starch (preferably potato or tapioca starch), alginic acid and certain complex silicates, together with binding agents such as polyvinylpyrrolidione, sucrose, gelatin and acacia. Additionally, lubricating agents such as magnesium stearate, sodium lauryl sulfate and talc are often very useful for tabletting purposes. Solid compositions of a similar type may also be employed as fillers in soft and hard-filled gelatin capsules; preferred materials in this connection would also include lactose or milk sugar as well as high molecular weight polyethylene glycols. When aqueous suspensions and/or elixirs are desired for oral administration, the essential active ingredient therein may be combined with various sweetening

or flavoring agents, coloring matter or dyes and, if so desired, emulsifying and/or suspending agents as well, together with such diluents as water, ethanol, propylene glycol, glycerin and various like combinations thereof.

## EXAMPLE 1

### 2-(2-Cyclohexyl)-1-{Dihydro-4-(2-Methyl-2-propyl)-5-Oxo-2H-Furan-2-yl}-1-{3-[4(5)-Imidazolyl]-2-(3-Indolecarbonyl)amino-Propionamido}Ethane

A mixture of indole-3-carboxylic acid (67.8 mg, 0.4205 mM), N-methylmorpholine (85.1 mg, 92.5 ml, 0.8410 mM), 2-(2-cyclohexyl)-1-[1-amino-2-(4(5)-imidazolyl)propionamido]-1-[dihydro-4-(2-methylpropyl)-5-oxo-2H-furan-2-yl]ethane (201 mg, 0.4205 mM), 1-hydroxybenzotriazole (56.0 mg, 0.4205 mM), and 1,3-dicyclohexylcarbodiimide (86.6 mg, 0.4205 mM) in methylene chloride (20 ml) was stirred at room temperature for 40 hours. It was then filtered and the filtrate stripped under reduced pressure. The residue was taken up in ethyl acetate, the resulting suspension filtered and the filtrate washed successively with water (1 x 20ml), saturated aqueous sodium bicarbonate solution (1 x 20ml) and brine (1 x 20ml), then dried over magnesium sulfate. Evaporation of the solvent under reduced pressure afforded 198 mg of crude title product as a foam.

The foam was chromatographed on a silica gel column, eluting first with 99:1 chloroform:methanol (thirty 4 ml fractions being collected), then with 97.5:2.5 chloroform:methanol (120 x 4 ml fractions) and finally with 95:5 chloroform:methanol (220 x 4 ml fractions). Removal of the solvent under reduced pressure gave 36 mg of the title compound.

$^1$H-nmr($CDCl_3$)delta: 1.0 [d, J=5Hz, 6H, $(CH_3)_2$].

The following compounds were prepared in like manner from appropriate reactants:

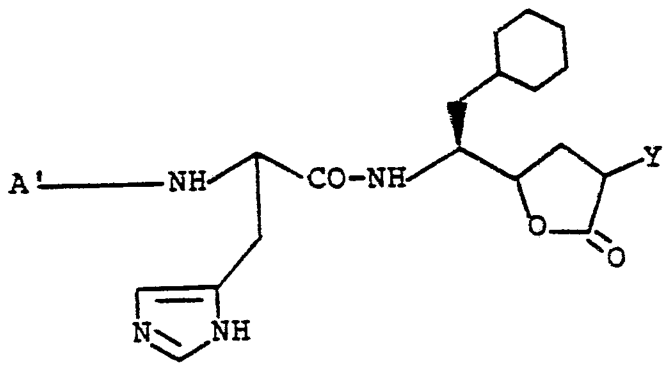

| A' | Y | $^1$H-nmr ($CDCl_3$) delta: |
|---|---|---|
| 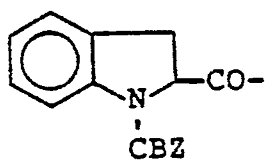 | $CH_2CH(CH_3)_2$ | 1.0 [d, J=5Hz, 6H, $(CH_3)_2$], 5.2 (s, 2H, benzylic methylene) |
| 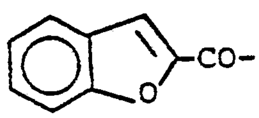 | $CH_2CH(CH_3)_2$ | 1.0 [d, J=5Hz, 6H, $(CH_3)_2$] |
| 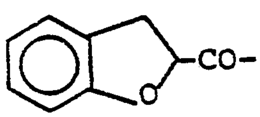 | $CH_2CH(CH_3)_2$ | 1.0 [d, J=5Hz, 6H, $(CH_3)_2$] |
| 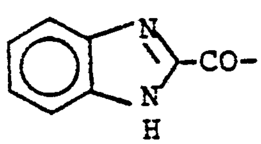 | $CH_2CH(CH_3)_2$ | 1.0 [d, J=5Hz, 6H, $(CH_3)_2$] |
| 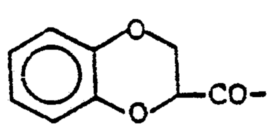 | $CH_2CH(CH_3)_2$ | 1.0 [d, J=5Hz, 6H, $(CH_3)_2$] |

| A' | Y | $^1H$-nmr ($CDCl_3$) delta: |
|---|---|---|
| 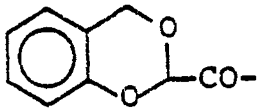 | $CH_2CH(CH_3)_2$ | 1.0 [d, J=5Hz, 6H, $(CH_3)_2$] |
| 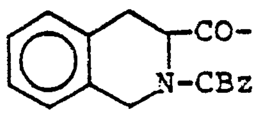 | $CH_2CH(CH_3)_2$ | 1.0 [d, J=5Hz, 6H, $(CH_3)_2$] |
| 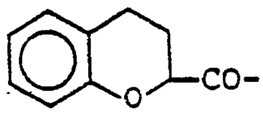 | $CH_2CH(CH_3)_2$ | 1.0 [d, J=5Hz, 6H, $(CH_3)_2$] |
| 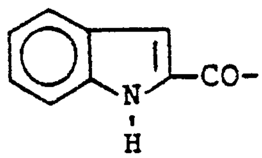 | $CH_2CH=CH_2$ | 4.8-5.2 (m, 2H, terminal methylene) |

## EXAMPLE 2

### t-Butyl-1-[2-Cyclohexyl-1-{Dihydro-4-Allyl-5-Oxo-2H-furan-2-yl}ethyl Carbamyl]-2-[1-t-Butoxycarbonyl-4(5)-Imidazolyl]ethyl Carbamate

DIBOCHis-ahcSta Lactone

A mixture of diBoc histidine (101 mg, 0.2845 mM), N-methylmorpholine (28.8 mg, 31.3 ml, 0.2845 mM), 1-hydroxybenzotriazole (38.4 mg, 0.2845 mM), 1,3-dicyclohexylcarbodiimide (59.1 mg, 0.2045 mM), 1-dihydro-4-allyl-2-[(1-amino)-2-cyclohexyl)ethyl]-5-oxo-2H-furanhydrochloride (42.5 mg, 0.2045 mM) in methylene chloride (20 ml) was stirred at room temperature overnight (18 hours) then filtered and the filtrate stripped under reduced pressure. The residue was taken up in ethyl acetate, the suspension filtered and the filtrate washed successively with 10% citric acid (1 x 20 ml), saturated aqueous sodium bicarbonate (1 x 20 ml) and brine

1 x 20 ml) and then dried over magnesium sulfate. Removal of the solvent gave 183 mg of title product as a foam.

$^1$H-nmr($CDCl_3$)delta: 1.5 (s, 9H, BOC), 1.7 (s, 9H, BOC).

DiBOCHis-ibhcSta Lactone

In like manner, diBOChistidine (355 mg, 1 mM) was reacted with 1-amino-2-cyclohexyl-1-[dihydro-4-(2-methylpropyl)-5-oxo-2H-furan-2-yl]ethane hydrochloride (304 mg, 1mM) to give 536 mg of condensation product diBOCHis-ibhcSta lactone.

$^1$H-nmr($CDCl_3$)delta: 1.5 (s, 9H, BOC), 1.7 (s, 9H, BOC).

EXAMPLE 3

N-Methyl-2R,4S,5S-[6-Cyclohexyl-4-Hydroxy-5-{3-[4(5)-Imidazolyl]}-2-{(3-Indolecarbonyl)amino} Propionamide-2-(2-Methylpropyl)]Hexanamide

or 3-Indolecarbonyl-His-ibhcSta-$NHCH_3$

The title product of Example 1 (36 mg) is dissolved in methanol (5 ml), the solution saturated with methylamine gas and stirred for 40 hours at room temperature. The reaction was stripped *in vacuo* to give 39 mg of the title compound as a yellow foam.

$^1$H-nmr($CDCl_3$)delta: 2.8 (s, 3H, aminomethyl).

The remaining compounds of Example 1 were converted mutatis mutandis to the following products by the above procedure:

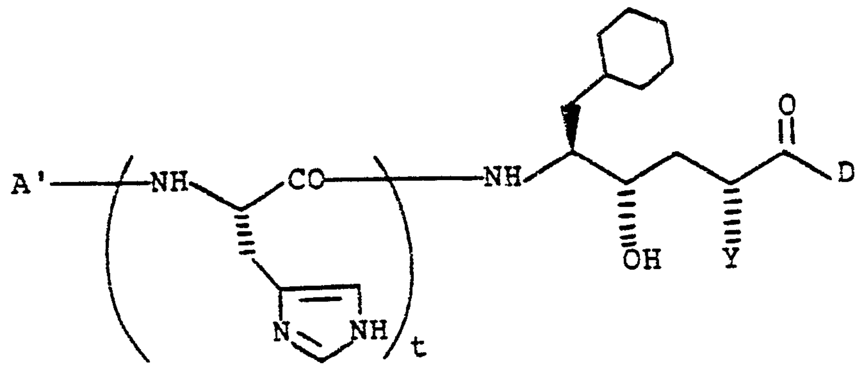

| A' | Y | D | t | $^1$H-nmr (CDCl$_3$) delta |
|---|---|---|---|---|
| 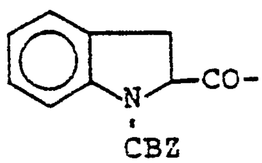 | $CH_2CH(CH_3)_2$ | $NHCH_3$ | 1 | 2.8 (s, 3H), 5.2 (s, 2H), (benzylic methylene) |
| 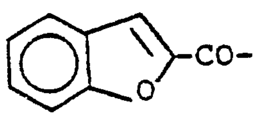 | $CH_2CH(CH_3)_2$ | $NHCH_3$ | 1 | 2.8 (s, 3H) |
| 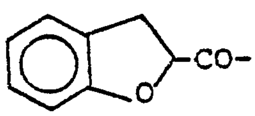 | $CH_2CH(CH_3)_2$ | $NHCH_3$ | 1 | 2.8 (s, 3H) |

| A' | Y | D | t | $^1H$-nmr $(CDCl_3)$ delta |
|---|---|---|---|---|
| 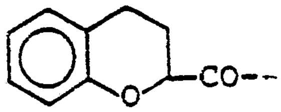 | $CH_2CH(CH_3)_2$ | $NHCH_3$ | 1 | 2.8 (s, 3H) |
| 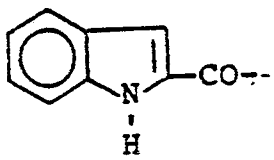 | $CH_2CH{=}CH_2$ | $NHCH_3$ | 1 | 2.8 (s, 3H) |
| 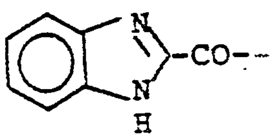 | $CH_2CH(CH_3)_2$ | $NHCH_3$ | 1 | 2.8 (s, 3H) |
| 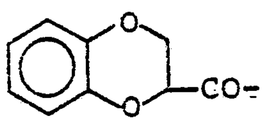 | $CH_2CH(CH_3)_2$ | $NHCH_3$ | 1 | 2.8 (s, 3H) |
| 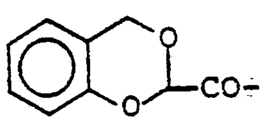 | $CH_2CH(CH_3)_2$ | $NHCH_3$ | 1 | 2.8 (s, 3H) |
| 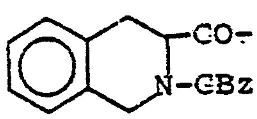 | $CH_2CH(CH_3)_2$ | $NHCH_3$ | 1 | 2.8 (s, 3H) |

EXAMPLE 4

N-Methyl-2R,4S,5S-5-Amino-6-Cyclohexyl-4-Hydroxy-2-(2-Methylpropyl)hexanamide Hydrochloride or ibhcSta-$NHCH_3HCl$

A mixture of N-methyl-6-cyclohexyl-4-hydroxy-2-(2-methylpropyl)-5-(t-butoxycarbonyl-amino)hexanamide (140 mg) and 4N HCl in dioxane (4 ml) was stirred at room temperature for 60 minutes. The reaction was then stripped under reduced pressure to give 187 mg of the deblocked product as a foam. $^1$H-nmr($CD_3OD$)delta: 2.8 (s, 3H).

The following compounds were prepared from appropriate BOC derivatives in like manner:

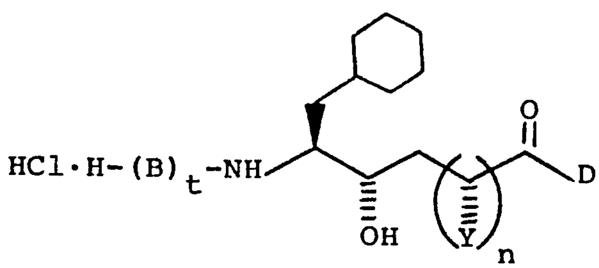

| t | B | n | Y | D | $^1$H-nmr ($CD_3OD$) delta |
|---|---|---|---|---|---|
| 0 | --- | 0 | --- | $NHCH_2CH(CH_3)C_2H_5$ | 0.9 (d, J=5Hz, 3H) |
| 0 | --- | 1 | $CH_2CH(CH_3)_2$ | $NHCH_3$ | 2.8 (s, 2H) |
| 0 | --- | 0 | --- | $NHCHCH_2OH$<br>$CH(CH_3)C_2H_5$ | 0.9 (d, J=5Hz, 3H) |
| 1[a] | His | 1 | $CH_2CH(CH_3)_2$ | $NHCH_2CH(CH_3)C_2H_5$ | 0.9 (d, J=5Hz, 3H) |
| 1 | Nle | 1 | $CH_2CH(CH_3)_2$ | $NHCH_3$ | 2.8 (s, 2H) |
| 1 | Ala | 0 | --- | $NHCH_2CH(CH_3)C_2H_5$ | 0.9 (d, J=5Hz, 3H) |
| 1 | Nle | 0 | --- | $NHCHCH_2OH$<br>$CH(CH_3)C_2H_5$ | 0.9 (d, J=5Hz, 3H) |
| 1 | Ala | 0 | --- | $NH(CH_2)_3COOBz$ | 5.2 (s, 2H) |
| 1[a] | His | 0 | --- | $NHCH_2CH(CH_3)C_2H_5$ | 0.9 (d, J=5Hz, 3H) |
| 1 | Ala | 0 | --- | $NHCH_2CH(CH_3)C_2H_5$ | 0.9 (d, J=5Hz, 3H) |

[a] Obtained as dihydrochloride salt

| t | B | n | Y | D | $^1$H-nmr ($CD_3OD$) delta |
|---|---|---|---|---|---|
| 1 | Ala | 1 | $CH_2CH(CH_3)_2$ | $NHCH_3$ | 2.8 (s, 2H) |
| 0 | --- | 1 | $CH_2CH(CH_3)_2$ | $NH(CH_2)_5NHCBz$ | 5.2 (s, 2H) |
| 1 | Ala | 1 | $CH_2CH(CH_3)_2$ | $NH(CH_2)_3COOBz$ | 5.2 (s, 2H) |
| 0 | His | 0 | --- | 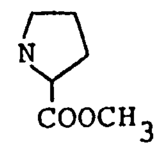 | 3.8 (s, 3H) |

## EXAMPLE 5

The compounds shown below were prepared from appropriate BOC derivatives according to the procedure of Example 4.

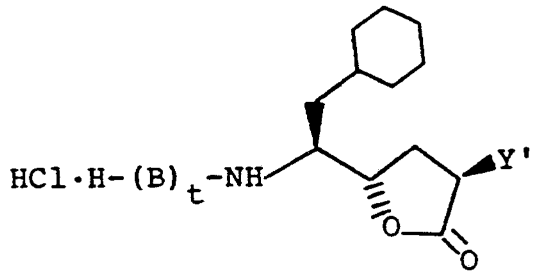

| t | B | Y' | $^1$H-nmr ($CD_3OD$) delta |
|---|---|---|---|
| 0 | --- | $CH_2CH(CH_3)_2$ | 1.0 (d, J=5Hz, 6H) |
| 0 | --- | $CH_2CH{=}CH_2$ | 4.8-5.2 (m, 2H) |
| 1[(a)] | His | $CH_2CH(CH_3)_2$ | 1.0 (d, J=5Hz, 6H) |
| 1[(a)] | His | $CH_2CH{=}CH_2$ | 4.8-5.2 (m, 2H) |
| 1[(a)] | His | H | 2.2 (m, 2H) |

[(a)] Obtained as dihydrochloride salts

## EXAMPLE 6

The compounds listed below were prepared from appropriate reactants according to the procedure of Example 1.

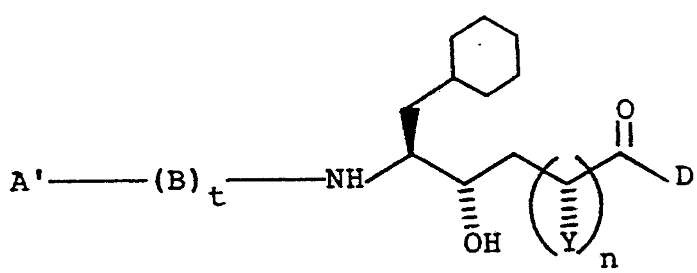

| A' | t | B | n | Y | D | Reaction | $^1$H-nmr (CDCl$_3$) delta |
|---|---|---|---|---|---|---|---|
| 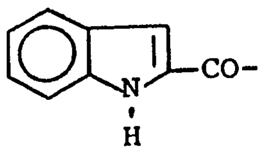 | 1 | His | 0 | --- | $NHCH_2CH(CH_3)C_2H_5$ | A'+B-C-D | 0.9 (d, J=5Hz, 3H) |
| 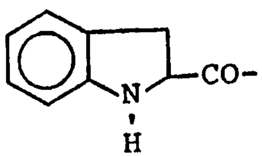 | 1 | Nle | 0 | --- | $NHCH-CH_2OH$<br>$CH(CH_3)C_2H_5$ | A'+B-C-D | 0.9 (d, J=5Hz, 3H) |
| --- | 1 | BOCNle | 0 | --- | $NHCH-CH_2OH$<br>$CH(CH_3)C_2H_5$ | B+C-D | 1.5 (s, 9H), 0.9 (d, J=5Hz, 3H) |
| --- | 1 | BOCNle | 1 | $CH_2CH(CH_3)_2$ | $NHCH_3$ | B+C-D | 1.5 (s, 9H), 2.8 (s, 2H) |
| --- | 1 | diBOCHis | 0 | --- | $NHCH_2CH(CH_3)C_2H_5$ | B+C-D | 1.5 (s, 9H), 1.7 (s, 9H) |
| --- | 1 | BOCAla | 1 | $CH_2CH(CH_3)_2$ | $NH(CH_2)_3COOBz$ | B+C-D | 1.5 (s, 9H) |

| A' | t | B | n | Y | D | Reaction | $^1$H-nmr ($CDCl_3$) delta |
|---|---|---|---|---|---|---|---|
| 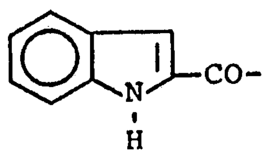 | 1 | Ala | 1 | $CH_2CH(CH_3)_2$ | $NH(CH_2)_3COOBz$ | A'+B-C-D | 5.2 (s, 2H) |
| --- | 1 | BOCAla | 0 | --- | $NHCH(CH_3)C_2H_5$ | B+C-D | 0.9 (d, J=5Hz, 3H) |
| 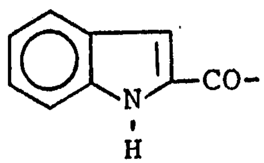 | 1 | Ala | 0 | --- | $NHCH(CH_3)C_2H_5$ | B+C-D | 0.9 (d, J=5Hz, 3H) |
| 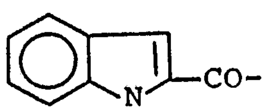 | 1 | BOCHis | 1 | $CH_2CH(CH_3)_2$ | $NH(CH_2)_5NHCBz$ | A'-B+C-D | 5.2 (s, 2H) |
| 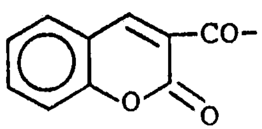 | 1 | Nle | | $CH_2CH(CH_3)_2$ | $NHCH_3$ | A'+B-C-D | 2.8 (s, 2H) |

| A' | t | B | n | Y | D | Reaction | $^1$H-nmr ($CDCl_3$) delta |
|---|---|---|---|---|---|---|---|
| BOC | 0 | --- | 0 | --- | NHCH-$CH_2$OH, CH$(CH_3)_2C_2H_5$ | C+D | 0.9 (d, J=5Hz, 3H) |
| BOC | 0 | --- | 0 | --- | $NHCH_2CH(CH_3)C_2H_5$ | C+D | 1.5 (s, 9H) |
| 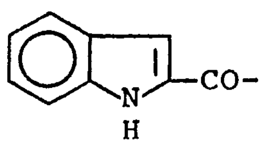 | 1 | BOCHis | 1 | $CH_2CH(CH_3)_2$ | $NH(CH_2)_5NHCBz$ | A'+B-C-D | 5.2 (s, 2H) |
| | | DiBOCHis | 0 | --- | 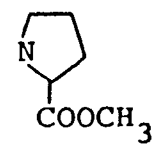 | B+C-D | 3.8 (s, 3H) |
| 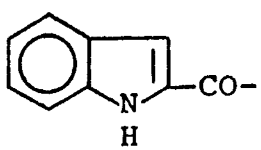 | 1 | His | 0 | --- | 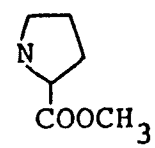 | A'+B-C-D | 3.8 (s, 2H) |

EXAMPLE 7

N-Methyl-2R,4S,5S-[6-Cyclohexyl-4-Hydroxy-5-{3-[4(5)-Imidazolyl]-2-(2-Indolecarbonylamino)-propionamido}-2-(2-Methylpropyl)]hexanamide

or [2-(2-Indolinecarbonyl)-His-ibhcSta-$NHCH_3$]

A mixture of 2-(2-(1-carbobenzoxy)indoline carbonyl)-His-ibhcSta-$NHCH_3$ (60 mg) and 10% palladium hydroxide-on-charcoal (35 mg) in methanol (10 ml) was hydrogenated at 3.52 kg/$cm^2$ (50 psi) and ambient temperature for three hours in a Paar shaker. The contents were then removed from the shaker, filtered through diatomaceous earth and the filtrate stripped *in vacuo*.

NMR of the residue showed the presence of the CBz group. The reduction and stripping steps were repeated two times using fresh catalyst each time to give 53 mg of the deblocked compound as a glass.
$^1$H-nmr($CDCl_3$)delta: 2.8 (s, 3H).

In like manner, following the procedure of the first paragraph but using a reaction period of 18 hours, [2-(2-carbobenzoxy-3-isoquinolinecarbonyl)-His-ibhcSta-$NHCH_3$ was deblocked. $^1$H-nmr($CDCl_3$)delta of the product: 2.8 (s, 3H).

EXAMPLE 8

2R,4S,5S-4-[6-Cyclohexyl-4-Hydroxy-5-{(2-[2-Indolecarbonyl]amino)propionamido}-2-(2-Methylpropyl)hexanamido]butanoic Acid

or 4-[2-(2-Indolecarbonyl)-Ala-ibhcSta]GABA

A mixture of the benzyl ester of 4-[2-(2-indolecarbonyl)-Ala-ibhcSta]GABA (36 mg) and 10% palladium/C (20 mg) in methanol (10 ml) was hydrogenated at 3.52 kg/$cm^2$ (50 psi) and room temperature for one hour.

The reaction mixture was then filtered through diatomaceous earth and stripped *in vacuo* to afford 26 mg of title product as a glass.
$^1$H-nmr($CDCl_3$)delta: 1.0 (d, J=5Hz, 6H).

Similarly, N-(5-aminopentyl)-[6-cyclohexyl-4-hydroxy-5{3-[4(5)-imidazolyl]-2-(2-indolecarbonylamino)propionamido}-2-(2-methylpropyl)]hexanamide was prepared from the benzyloxycarbonyl compound of Example 9.
$^1$H-nmr($CDCl_3$)delta: 1.0 (d, J=Hz, 6H).

## EXAMPLE 9

### N-(5-Benzyloxycarbonylaminopentyl-2R,4S,5S-[6-Cyclohexyl-4-Hydroxy-5-{3-[4(5)-Imidazolyl]-2-(2-Indolecarbonylamino)-Propionamide}-2-(2-Methylpropyl)]hexanamide

To a solution of N-(5-benzyloxycarbonylaminopentyl)-[6-cyclohexyl-4-hydroxy-5- 3-(1-t-butoxycarbonyl)-[4(5)-imidazolyl]-2-(2-indolecarbonylamino)propionamido -2-(2-methylpropyl)]hexanamide (38.3 mg, 0.0456 mM) in methanol (2 ml) at room temperature was added a catalytic amount of potassium carbonate (about 10% by weight of BOC derivative). The reaction is stirred for 3 hours, the methanol removed under reduced pressure and ether added to the residue. Evaporation of the ether afforded a gummy residue which was dissolved in minimal volume of dioxane, the solution transferred to a vial and freeze-dried to give 33 mg of title product.
$^1$H-nmr($CDCl_3$)delta: 1.0 (d, J=5Hz, 6H).

## EXAMPLE 10

**N-(3-Benzyloxycarbonylpropyl)-2R,4S,5S-[6-Cyclohexyl-4-Hydroxy-2-(2-Methylpropyl)-5-{2-Methyl-2-(t-Butylcarbonylamino)ethyl}]Hexanamide**

or BOCAla-ibhcSta-NH$(CH_2)_3CO_2$Bz

To a solution of the product of Preparation 18 (277 mg, 0.5572 mM) and BOCAla (127 mg, 0.6686 mM) in methylene chloride (5 ml) at 0° C. was added diethyl cyanophosphonate (0.104 ml, 0.6686 mM) followed by N-methylmorpholine (0.147 ml, 1.337 mM) and the reaction stirred at 0° C. for one hour, then at room temperature for 4 hours. The solvent was removed under reduced pressure, the residue taken up in ethyl acetate (20 ml) and the solution washed successively with 5% HCl (2 x 20 ml), water (1 x 20 ml), saturated sodium carbonate solution (2 x 20 ml), water (1 x 20 ml) and brine (1 x 20 ml), then dried ($MgSO_4$). Evaporation of the solvent under reduced pressure gave 248 mg of crude title product which was purified by chromatography on 3.0 g of fine mesh silica gel using 97.5:2.5 chloroform:methanol as eluant. Yield 166 mg of title product. $^1$H-nmr($CDCl_3$)delta: 5.2 (s, 2H).

## PREPARATION 1

## S-3-Cyclohexyl-2-(t-butoxycarbonylamino)propionaldehyde

Methyl S-3-cyclohexyl-2-(t-butoxycarbonylamino)-propionate (51.2 g, 0.179 mol) was dissolved in 728 ml of dry toluene and cooled to -78° C. Diisobutylaluminum hydride (449 ml of 1M in toluene, 0.449 mol) was added dropwise over 1 hour, maintaining -70° to -78° C. Methanol (13 ml) was added at -70° C., followed by 608 ml of half-saturated sodium potassium tartrate, and the mixture warmed to ambient temperature. Ether (300 ml) was added and the organic layer was separated and washed with 1 liter saturated sodium potassium tartrate. The original aqueous layer was extracted with 600 ml fresh ether and backwashed with 600 ml fresh saturated sodium potassium tartrate. The organic layers were combined, dried over $MgSO_4$ and stripped to yield title product as a gum, contaminated with toluene on the basis of $^1$H-nmr, 45.6 g; tlc Rf 0.45 (1:3 ethyl acetate:hexane); $^1$H-nmr($CDCl_3$)delta: 0.9 to 2.3 (m), which includes t-butyl singlet at 1.4, 3.0-4.8 (m), 4.9-5.2 (d), 9.6 (s).

## PREPARATION 2

## Ethyl 4RS,5S-6-Cyclohexyl-5-(t-butoxycarbonylamino)-4-hydroxy-2-hexynoate

Dry freshly distilled THF (117 mol) and diisopropylamine (22.0 ml, 15.8 g, 0.156 ml) were charged to a flame dried reaction flask under $N_2$ and the resulting solution cooled to -30° C. and butyllithium (76.9 ml of 1.6M in hexane, 0.123 mol) added over 5 minutes. The solution was then cooled to -78° C. and ethyl propiolate (12.5 ml, 12.1 g, 0.123 mol) added dropwise over 20 minutes, maintaining the temperature -65° to -78° C.

After 30 minutes at -78° C., title product of the preceding Preparation (19.52 g, 0.0866 mol) in 35 ml THF was added over 20 minutes, again maintaining -65° to -78° C. After 2 hours, 200 ml of 5:1 THF:acetic acid was added to the reaction mixture, and it was allowed to warm to ambient temperature and diluted with a half volume of ether and an equal volume of 10% citric acid.

The organic layer was separated, washed sequentially with 2 x 200 ml fresh 10% citric acid, 200 ml of brine and 2 x 200 ml saturated $NaHCO_3$, dried over $MgSO_4$ and stripped to a dark red oil, 38.2 g. The latter was chromatographed on a 10 cm x 42 cm column of silica gel with tlc monitoring, eluting with 5 liters of 1:9 ethyl acetate:hexane. After 1500 ml to develop the column, 500 ml fractions were collected. Fractions 29-37 were combined and stripped to yield title product as an oil, 15.3 g; tlc Rf 0.44 (3:7 ethyl acetate:hexane); $^1$H-nmr($CDCl_3$)delta: 1.0-2.0 (m, 25H) including singlet for the t-butyl group at 1.5, 3.8-5 (m, 6H).

PREPARATION 3

t-Butyl[2-cyclohexyl-1-(dihydro-5-oxo-2H-furan-2-yl)ethyl]carbamate

Title product of the preceding Preparation (18.28 g) and 5% Pd/$BaSO_4$ (10.97 g) were combined with 150 ml ethyl acetate and hydrogenated for 2 hours under 4 atmospheres pressure of hydrogen. The catalyst was recovered by filtration and the filtrate stripped to yield intermediate ethyl 4RS,5S-6-cyclohexyl-4-hydroxy-5-(t-butyloxyamino)hexanoate, 19 g. The latter was taken up in 250 ml of 2.5% acetic acid in toluene, refluxed 2.5 to 3 hours, stripped and the residue chromatographed on a 10 cm x 30 cm column of silica

gel, monitoring by tlc, eluting with 4 liters of 9:11 ether:hexane, 8 liters of 1:1 ether:hexane, 2 liters of 11:9 ether:hexane and finally 3 liters of 3:2 ether: hexane, collecting 28 x 400 ml fractions, 6 x 150 ml fractions and finally 11 x 400 ml fractions. Fractions 17-24 were combined and co-stripped with ether to yield the predominant and desired, less polar, 4S,5S-title product as an oil, 9.13 g; tlc Rf 0.5 (7:3 ether: hexane). The more polar, 4R,5S-epimer of title product was isolated by stripping combined fractions 28-45 and crystallized by trituration with hexane, 1.77 g; m.p. 101.5-103.5° C.

PREPARATION 4

t-Butyl[2-cyclohexyl-1-{dihydro-4-(2-methyl-2-propenyl)-5-oxo-2H-furan-2-yl}ethyl]carbamate

Dry freshly distilled THF (30 ml) and diisopropylamine (3.51 ml, 2.52 g., 0.0249 mol) were charged to a flame dried reaction flask under $N_2$, the resulting solution was cooled to -50° C., butyllithium (13.9 ml of 1.6M in hexane, 0.0222 mol) was added and the mixture further cooled to -78° C. Title product of the preceding Preparation (2.77 g, 0.0089 mol) in 15 ml THF was added dropwise over 10 minutes and the enolate allowed to form over a further 20 minutes at -78° C., at which time 3-bromo-2-methyl-1-propene in 5 ml THF was added over 10 minutes, and the mixture stirred an additional 1 hour at -78° C., quenched with 5 ml saturated $NH_4Cl$, warmed to room temperature, diluted with a half volume of ether, washed 2 x 50 ml 10% citric acid, 2 x 50 ml saturated $NaHCO_3$ and 1 x 25 ml brine, dried over $MgSO_4$ and stripped to an oil, 3.06 g, a mixture of the title epimers. The latter were separated by chromatography on 7 cm x 20 cm silica gel;

monitoring by tlc; eluting sequentially with 2 liters of 1:9 ether:hexane, 4 liters of 3:17 ether:hexane, 2 liters of 1:4 ether:hexane, 2 liters of 1:3 ether:hexane, 2 liters of 7:13 ether: hexane and 2 liters of 1:1 ether:hexane; and collecting 125 ml fractions. The less polar title product, having trans (2R) stereochemistry, was collected in fractions 30-48, combined and stripped to yield same as an oil, 1.17 g; tlc Rf 0.45, (2:3 ether:hexane); $^1$H-nmr($CDCl_3$)delta 1.4 (s, 9H), 1.8 (s, 3H), 0.3-3.0 (m, 18H), 3.6-4.0 (m, 1H), 4.69 (s, 1H), 4.75 (s, 1H), 4.1-4.8 (m, 2H). Fractions 55-76 gave the more polar title product, also as an oil, 0.358 g, having cis (2S) stereochemistry; tlc Rf 0.36 (2:3 ether:hexane); $^1$H-nmr identical to that of the less polar epimer.

PREPARATION 5

t-Butyl[2-cyclohexyl-1-{dihydro-4-(2-methylpropyl)-5-oxo-2H-furan-2-yl}ethyl]carbamate

The less polar title product of the preceding Preparation (1.17 g) and 10% Pd/C (0.351 g) were combined in 20 ml ethyl acetate and hydrogenated at 4 atmospheres pressure for 2.5 hours, the catalyst recovered by filtration and the filtrate stripped to yield less polar title product (likewise having trans, i.e., 2R stereochemistry) as an oil which crystallized on standing, 1.20 g; m.p. 88-93° C.; tlc Rf 0.65 (1:1 ether:hexane), Rf 0.73 (2:1 ethyl acetate:hexane). The other isomer, having cis (2S) stereochemistry, was obtained in like manner; tlc Rf 0.59 (1:1 ether:hexane). In subsequent Preparations, which employ the present less polar epimer of Rf 0.65 (1:1 ether:hexane), 2R stereochemistry is specified.

PREPARATION 6

Benzyl 2R,4S,5S-6-Cyclohexyl-5-(t-butoxycarbonylamino)-4-hydroxy-2-(2-methylpropyl)hexanoate

The less polar 2R title product of the preceding Preparation (1.0 g, 2.72 mmol), DME (14 ml) and NaOH (4.4 ml of 5N, 0.022 mol) were combined, stirred for 12 hours and then stripped of organic solvent. The aqueous residue was diluted with water (5 ml), layered with an equal volume of ether, cooled to 0° C. and acidified with 22 ml of 1N HCl, by which time the aqueous layer was turbid. The ether layer was separated and the aqueous layer (pH 2.0) washed with an equal volume of fresh ether. The ether layers were combined, dried over $MgSO_4$ and stripped to yield intermediate 2R,4S,5S-6-cyclohexyl-5-(t-butoxycarbonylamino)-4-hydroxy-2-(2-methylpropyl)hexanoic acid as a white foam, 0.909 g (2.36 mmol). The latter was charged into 20 ml DMF together with $K_2CO_3$ (0.330 g, 2.36 mmol) and cooled to 0° C. Benzyl bromide (0.308 ml, 0.443 g, 2.59 mmol) was added and the stirred mixture allowed to warm to room temperature. After 4 hours, the reaction mixture was diluted with an equal volume of ether, washed 1 x 75 ml 10% citric acid, 1 x 75 ml saturated $NaHCO_3$, dried over $MgSO_4$, and stripped to yield an oil, 1.35 g. The latter was chromatographed on 4 cm x 20 cm silica gel; monitored by tlc; eluting with 1 liter 1:9 ether:hexane, 1 liter 3:17 ether:hexane, 1 liter 1:4 ether:hexane and finally 1 liter 3:7 ether:hexane; and collecting 23 ml fractions. Fractions 46-75 were combined and stripped to yield crystalline starting material, 0.583 g, suitable for recycling. Fractions 85-119 were combined and stripped to yield present

title product as an oil, 0.275 g; tlc Rf 0.47 (1:1 ether:hexane);
$^1$H-nmr($CDCl_3$): 0.3-2.0 (m, 24H), 1.4 (s, 9H), 2-2.4 (m, 1H), 2.6-3.0 (m, 1H), 3.2-3.7 (m, 2H), 4.65 (d, 1H), 5.1 (s, 2H), 7.3 (s, 5H).

PREPARATION 7

Benzyl 2R,4S,5S-6-Cyclohexyl-5-(t-butoxycarbonylamino)-2-(2-methylpropyl)-4-(2-tetrahydropyranyloxy)hexanoate

The product of the preceding Preparation (0.442 g, 0.929 mmol), 3,4-dihydro-2H-pyran (97%, 0.255 ml, 0.235 g, 2.79 mmol) and pyridinium p-tosylate (23 mg, 0.093 mmol) were combined in 6.5 ml $CH_2Cl_2$ and stirred in a stoppered flask for 18 hours, then stripped of solvent and the residue taken up in 100 ml ether, washed 2 x 100 ml half saturated brine, dried over $MgSO_4$, co-stripped with $CH_2Cl_2$, and dried under high vacuum to yield present title product as an oil, 0.565 g; tlc Rf 0.59 (2:3 ether:hexane).

PREPARATION 8

2R,4S,5S-6-Cyclohexyl-5-(t-butoxycarbonylamino)-2-(2-methylpropyl)-4-(2-tetrahydropyranyloxy)hexanoic Acid

The product of the preceding Preparation (0.565 g) and 10% Pd/C (0.170 g) were combined in 10 ml ethyl acetate and hydrogenated at 4 atmospheres pressure for 2.5 hours. The catalyst was recovered by filtration and the filtrate co-stripped with $CH_2Cl_2$ to yield title product as an oil, 0.55 g, which contains the theoretical yield (0.474 g) of product; tlc Rf 0.0 (2:3 ether:hexane), 0.35 (1:1 ethyl acetate:hexane); $^1$H-nmr($CDCl_3$) includes 9.5-10.1 (bs, 1H, COOH) and no peaks due to a benzyl group.

This Preparation was repeated on a 300 mg scale with drying of the product under high vacuum to produce 250.5 mg (a quantitative yield of title product as a white foam).

PREPARATION 9

2R,4S,5S-6-Cyclohexyl-5-(t-butoxycarbonylamino)-4-hydroxy-2-(2-methylpropyl)-N-methylhexanamide

2R,4S,5S-6-Cyclohexyl-5-(t-butoxycarbonylamino)-2-(2-methylpropyl)-gamma-hexanolactone (197 mg, 0.536 mmol, Preparation 5) was dissolved in 1 ml of water and cooled in an ice-water bath. The cold solution was perfused with $CH_3NH_2$ for 3 minutes, stoppered and allowed to stand at room temperature for 2 hours, by which time tlc indicated reaction was complete. The mixture was stripped of solvent and dried under high vacuum to yield title product as a white, solid foam, 204 mg; tlc Rf 0.31 (3:1 ethyl acetate:hexane).

PREPARATION 10

2R,4S,5S-6-Cyclohexyl-5-amino-4-hydroxy-2-(2-methylpropyl)-N-methylhexanamide Hydrochloride

Title product of the preceding Example (199 mg, 0.499 mmol) was dissolved in 4N HCl in dioxane (1 ml) and stirred for 25 minutes under $N_2$, by which time tlc indicated complete consumption of starting material. The reaction mixture was then stripped of solvent and dried in high vacuum to produce title product as an incompletely dry, pale yellow powder, 185 mg; tlc Rf 0.20 (18:2:1 $CHCl_3$:ethanol:acetic acid; the tlc plate was exposed to $NH_3$ vapor prior to elution to convert the HCl salt to free base).

PREPARATION 11

t-Butyl[2-cyclohexyl-1-{dihydro-4-benzyl-5-oxo-2H-furan-2yl}ethyl]carbamate

Under $N_2$, dry di(isopropyl)amine (2.48 ml, 0.0177 mol; distilled from $CaH_2$) in dry THF (7.4 ml, distilled from K) was cooled to 0° C. Butyllithium (11.0 ml of 1.62M in hexane, 0.0177 mol) was added dropwise over 5 minutes. After stirring 15 minutes at 0° C., the mixture was cooled to -78° C. and the less polar 4S,5S-title product of Preparation 3 (2.30 g, 0.0074 mol) in 3.7 ml dry THF added over 5 minutes. After stirring 30 minutes more at -78° C., benzyl bromide (0.923 ml, 0.0078 mol) in 3.7 ml dry THF was added dropwise over 5 minutes. After 1 hour at -78° C., the reaction was quenched by the addition of 10 ml saturated $NH_4Cl$, warmed to room temperature, diluted with 25 ml ether and the layers separated. The organic layer was washed 2 x 15 ml saturated $NaHCO_3$, dried ($MgSO_4$) and stripped to an oil (3.29 g). The oil was chromatographed on 150 g silica gel eluting with 2.5 liters 1:9 ethyl acetate:hexane and monitoring by tlc. Clean product fractions were combined and stripped to yield purified title product as a white oily solid, 1.46 g; tlc Rf 0.60 (1:1 ethyl acetate:hexane). More polar starting material (0.91 g of yellow oil about 70% pure, tlc Rf 0.4 in the same system) was also recovered from the column.

PREPARATION 12

2R,4S,5S-6-Cyclohexyl-5-(t-butoxycarbonylamino)-4-hydroxy-2-benzyl-N-methylhexanamide

Title product of the preceding Preparation (157 mg) was dissolved in 2 ml $CH_3OH$, cooled to 0° C., and perfused with $CH_3NH_2$ for 3 minutes. The flask was stoppered, allowed to stand at room temperature for 1.5 hours, stripped with 2 x 2 ml ether chase and dried

under high vacuum to yield title product as a white, solid foam, 164 mg; tlc Rf 0.17 (1:1 ethyl acetate: hexane), Rf 0.29 (3:1 ethyl acetate:hexane); $^1$H-nmr[$CDCl_3$)300MHz showed the expected $C(CH_3)_3$] (s, 9H), N-$CH_3$ (d, 3H), aromatic resonances and t-butoxycarbonyl-NH (d, 1H), peaks.

PREPARATION 13

2R,4S,5S-6-Cyclohexyl-5-amino-4-hydroxy-2-benzyl-N-methylhexanamide Hydrochloride

The product of the preceding Preparation (159 mg) was dissolved in 2 ml 4N HCl in dioxane, stirred under $N_2$ 0.5 hour, stripped, chased 3 x 2 ml ether and the residue dried under high vacuum to yield title product as a pale yellow powder, 135 mg; tlc 0.20 (18:2:1 $CHCl_3$:ethanol:acetic acid; spotted plate exposed to $NH_3$ to neutralize HCl salt prior to elution).

PREPARATION 14

2R,4S,5S-6-Cyclohexyl-5-amino-4-hydroxy-2-(cyclohexylmethyl)-N-methylhexanamide Hydrochloride

By the method of Preparation 13, title product of the preceding Preparation (75 mg) was converted to present title product, 64.1 mg; tlc Rf 0.18 (18:2:1 $CHCl_3$:ethanol:acetic acid; spotted plate neutralized with $NH_3$ vapor before elution).

PREPARATION 15

N-Carbobenzoxy-indoline-2-carboxylic Acid

A solution of benzyl chloroformate (1.05 ml, 1.254 g, 7.353 mM) in tetrahydrofuran (THF, 5 ml) was slowly added to a solution of indoline-2-carboxylic acid (1.00 g, 6.127 mM) and sodium bicarbonate (514 mg, 6.127 mM) in THF:water (20 ml of 1:1) at 0-5° C. The pH was maintained at about 8.0 by addition of 1N NaOH

solution as needed. A total of 6.13 ml of 1N NaOH was used, the pH being stabilized at about 8.13. The reaction mixture was allowed to warm to room temperature and then stripped of THF. The aqueous residue was washed with ether and the ether layer removed. The aqueous layer was extracted with ethyl acetate at pH 2.0 (adjusted by 1N HCl). The ethyl acetate extraction was repeated and the combined ethyl acetate extracts washed with brine, then dried ($MgSO_4$). Stripping of the solvent afforded 1.46 g of product.

$^1H$-nmr($CDCl_3$)delta: 5.2 (s, 2H).

In like manner N-carbobenzoxyisoquinoline-3-carboxylic acid is prepared.

$^1H$-nmr($CDCl_3$)delta: 5.2 (s, 2H).

PREPARATION 16

t-Butyl[2-Cyclohexyl-1-{dihydro-4-allyl-5-oxo-2H-furan-2-yl}ethyl]carbamate

To a solution of diisopropylamine (928 mg, 129 ml, 9.1715 mM) in THF (30 ml) cooled to -40° C. was added n-butyllithium (5.12 ml of 1.6M in hexane, 8.1888 mM) dropwise. The reaction mixture was then cooled to -75° C. and t-butyl[2-cyclohexyl-1-{dihydro-5-oxo-2H-furan-2-yl}ethyl]carbamate (1.02 g, 3.2755 mM) in THF, 10 ml) added. The reaction mixture was stirred for 30 minutes at -75° C. at which time allyl bromide (476 mg, 340 µl, 3.9306 mM) was added dropwise, the temperature being maintained at between -75° and -65° C. Following addition of the allyl bromide, the reaction was stirred for 2 hours at -75° C. Additional allyl bromide (170 µl, 288 mg) was added at -75° C. and the reaction stirred for 2.5 hours. Ammonium chloride (5 ml of saturated aqueous solution) was then added dropwise and the reaction allowed to warm to room temperature. The THF was stripped off, the residue

taken up in ether and the ethereal solution washed successively with 10% aqueous citric acid solution, saturated sodium bicarbonate solution and brine and then dried ($MgSO_4$). Removal of solvent under reduced pressure gave 1.04 g of the title compound.

It was purified by chromatography on a silica gel column, eluting with hexane:ethyl acetate, 97.5:2.5 (140 x 12 ml fractions), hexane:ethyl acetate, 95:5 (170 x 12 ml fractions) and hexane:ethyl acetate, 9:1 (50 x 12 ml fractions). Removal of solvent from the last 55 fractions gave 372 mg of pure title compound. $^1$H-nmr($CDCl_3$)delta: 4.8-5.2 (m, 2H).

PREPARATION 17

2R,4S,5S,6-Cyclohexyl-5-(t-butoxycarbonyl-amino-4-(2-tetrahydropyranyloxy)-2-(2-methylpropyl)-N-(3-carbobenzoxypropyl)hexanamide or BOC-ibhcSta-GABA Benzyl Ester

To a solution of benzyl 4-aminobutanoate hydrochloride (118 mg, 0.7026 mM), 2R,4S,5S,6-cyclohexyl-5-t-butoxycarbonylamino-4-(2-tetrahydropyranyloxy)-2-(2-methylpropyl)hexanoic acid (300 mg, 0.638 mM) in methylene chloride (5 ml) at 0° C. was added diethyl cyanophosphonate (114.6 mg, 0.109 ml, 0.7026 mM), followed by N-methylmorpholine (0.147 ml, 1.34 mM). The reaction was stirred at 0° C. for one hour and then at room temperature for 4 hours. The methylene chloride was then removed under reduced pressure and ethyl acetate (20 ml) added to the residue. The resulting solution was washed successively with 5% HCl (2 x 20 ml), water (1 x 20 ml), saturated aqueous bicarbonate solution (2 x 20 ml), water (1 x 20 ml) and brine (1 x 20 ml), then dried over $MgSO_4$. Removal of the solvent under reduced pressure gave 385 mg of crude title product which was used without further purification. $^1$H-nmr($CDCl_3$)delta: 1.5 (s, 9H).

PREPARATION 18

2R,4S,5S,5-Amino-6-cyclohexyl-4-hydroxy-2-(2-methylpropyl)-N-carbobenzoxypropyl)hexanamide Hydrochloride or ibhcSta - GABA Benzyl Ester

The product of the preceding Preparation (359 mg, 0.5507 mM) in acetic acid (8 ml) and water (2 ml) was stirred for 16 hours and the reaction then evaporated under high vacuum at ambient temperature to give the de(2-tetrahydropyranyl) product.

The residue was dissolved in ether (3 ml), 4.7 N HCl/dioxane (4 ml) added at 0° C. and the reaction stirred for 4 hours. The volatiles were stripped under reduced pressure and the residue triturated with ether/hexane to give the title product as a white solid.

PREPARATION 19

Methyl 3-[4(5)-imidazolyl]-2-(2-indolecarbonylamino)propionate

To a solution of histidine methyl ester dihydrochloride (5.50 g, 22.7 mM) in methylene chloride (225 ml) at 0° C. was added triethylamine (6.92 ml, 49.8 mM) and the reaction stirred for 30 minutes at 0° C. Indole-2-carboxylic acid (4.2 g, 26.0 mM), 1-hydroxybenzotriazole (5.56 g, 36.3 mM) and 1,3-dicyclohexylcarbodiimide (5.35 g, 26.0 mM) were then added to the reaction in the order listed (under a nitrogen atmosphere) at 0° C. Stirring was continued at 0° C. for three hours after which the reaction was allowed to warm to room temperature, and stirring continued for 18 hours. Water (100 ml) was added to the reaction, the mixture filtered and then concentrated *in vacuo*. Addition of ethyl acetate (200 ml) to the concentrate gave a gummy yellow solid. The ethyl acetate was stripped off, chloroform (200 ml) added and the mixture

extracted with 1N NaOH solution (4 x 30 ml), brine (2 x 20 ml), then dried over $Na_2SO_4$. Concentration of the dried extract afforded 7.2 g (102%) of yellow foam.

Water (20 ml) and 1N HCl (25 ml) were added to the yellow foam and the solution extracted with chloroform (2 x 5 ml). The aqueous phase was adjusted to pH 6.0 and chilled overnight. The water was removed in vacuo and the residue azeotroped with toluene to give 4.3 g of solid comprising 25% salt as determined by HPLC in the system 35:65 acetonitrile:0.1M $PO_4^{-3}$, pH 2.1 and 75% of title product.

$^1$H-nmr($CDCl_3$)delta: 3.8 (s, 3H).

PREPARATION 20

1-(t-Butoxycarbonyl)-3-[4(5)-imidazolyl]-2-(2-indolecarbonylamino)propionic Acid

The product of Preparation 19 (4.3 g) was added to methanol (60 ml) and then water (20 ml) added. The solution was cooled to 0° C., potassium carbonate (7.6 g) added and the reaction stirred for one hour at 0° C. It was warmed to room temperature and stirred for two hours. The reaction was cooled to 10° C., adjusted to pH 3 by addition of 6N HCl, then stripped of methanol under reduced pressure. The aqueous solution remaining was cooled to 0° C., adjusted to pH 10.5 by addition of 6N NaOH solution, and dioxane (150 ml) added. Di-(t-butoxycarbonyl)oxide (3.9 g, 4.2 ml, 18 mM) was then added to the stirred reaction as a single portion and the reaction allowed to warm to room temperature. The pH of the reaction was maintained at 9-10.5 by addition of 6N NaOH as needed. After two hours of stirring, a second portion of di-(t-butoxycarbonyl)oxide (2 ml) was added to the reaction and stirring continued an additional 15 minutes. The

dioxane was then distilled off under reduced pressure, and water (100 ml) added to the residue which was then extracted with ether (3 x 25 ml). Crushed ice and ethyl acetate (500 ml) were added to the aqueous residue which was then adjusted to pH 1.3 by means of 6N HCl and vigorously stirred. The ethyl acetate phase was separated, the aqueous phase extracted with ethyl acetate (2 x 50 ml) and the combined extracts washed with brine (1 x 50 ml) and dried ($Na_2SO_4$). Removal of the ethyl acetate under reduced pressure gave 3.1 g (56%) of title product as a yellow powder.
$^1$H-nmr($CDCl_3$)delta: 1.7 (s, 9H).

PREPARATION 21

N-(5-Benzyloxycarbonylaminopentyl)-[6-cyclohexyl-2-(2-methylpropyl)-5-(t-butoxycarbonylamino)-4-(2-tetrahydropyranyloxy)]Hexanamide

The tetrahydropyranyl ether of BOChcSta (299 mg, 0.6367 mM), the mono CBz derivative of 1,5-diaminopentane hydrobromide (202 mg, 0.6367 mM), N-methylmorpholine (0.14 ml, 1.273 mM), diethylcyanophosphonate (0.10 ml, 0.6367 mM), and methylene chloride (5 ml) were combined at 0° C. and stirred for 3 hours. The temperature was then allowed to warm to room temperature and stirring continued overnight. The methylene chloride was removed under reduced pressure and ethyl acetate (20 ml) added to the residue. The ethyl acetate solution was washed successively with 2 x 10 ml of each of 5% HCl, water and saturated aqueous sodium bicarbonate. It was then dried over $MgSO_4$ and evaporated under reduced pressure to give 356 mg of crude product which was purified by column chromatography on a silica gel column using 99.5:5 chloroform:methanol as eluant. Yield of title product: 280 mg.
$^1$H-nmr($CDCl_3$)delta: 5.2 (s, 2H).

PREPARATION 22

t-Butyl[2-cyclohexyl-1- dihydro-4-benzyl-5-oxo-2H-furan-2-yl ethyl]carbamate

Under $N_2$, dry di(isopropyl)amine (2.48 ml, 0.0177 mol; distilled from $CaH_2$) in dry THF (7.4 ml, distilled from K) was cooled to 0° C. Butyllithium (11.0 ml of 1.62M in hexane, 0.0177 mol) was added dropwise over 5 minutes. After stirring 15 minutes at 0° C., the mixture was cooled to -78° C. and the less polar 4S,5S-title product of Preparation 3 (2.30 g, 0.0074 mol) in 3.7 ml dry THF added over 5 minutes. After stirring 30 minutes more at -78° C., benzyl bromide (0.923 ml, 0.0078 mol) in 3.7 ml dry THF was added dropwise over 5 minutes. After 1 hour at -78° C., the reaction was quenched by the addition of 10 ml saturated $NH_4Cl$, warmed to room temperature, diluted with 25 ml ether and the layers separated. The organic layer was washed 2 x 15 ml saturated $NaHCO_3$, dried ($MgSO_4$) and stripped to an oil (3.29 g). The oil was chromatographed on 150 g silica gel eluting with 2.5 liters 1:9 ethyl acetate:hexane and monitoring by tlc. Clean product fractions were combined and stripped to yield purified title product as a white oily solid, 1.46 g; tlc Rf 0.60 (1:1 ethyl acetate: hexane). More polar starting material (0.91 g of yellow oil about 70% pure, tlc Rf 0.4 in the same system) was also recovered from the column.

PREPARATION 23

2R,4S,5S-6-Cyclohexyl-5-(t-butoxycarbonyl-amino)-4-hydroxy-2-benzyl-N-methylhexanamide

Title product of the preceding Preparation (157 mg) was dissolved in 2 ml $CH_3OH$, cooled to 0° C. and perfused with $CH_3NH_2$ for 3 minutes. The flask was stoppered, allowed to stand at room temperature for 1.5

hours, stripped with 2 x 2 ml ether chase and dried under high vacuum to yield title product as a white, solid foam, 164 mg; tlc Rf 0.17 (1:1 ethyl acetate: hexane), Rf 0.29 (3:1 ethyl acetate:hexane); $^1$H-nmr[$CDCl_3$, 300MHz showed the expected $C(CH_3)_3$] (s, 9H), N-$CH_3$ (d, 3H), aromatic resonances and t-butoxycarbonyl-NH (d, 1H), peaks.

PREPARATION 24

2R,4S,5S-6-Cyclohexyl-5-amino-4-hydroxy-2-benzyl-N-methylhexanamide Hydrochloride

The product of the preceding Preparation (159 mg) was dissolved in 2 ml 4N HCl in dioxane, stirred under $N_2$ 0.5 hour, stripped, chased 3 x 2 ml ether and the residue dried under high vacuum to yield title product as a pale yellow powder, 135 mg; tlc 0.20 (18:2:1 $CHCl_3$:ethanol:acetic acid; spotted plate exposed to $NH_3$ to neutralize HCl salt prior to elution).

PREPARATION 25

Methyl[1-{5-cyclohexyl-3-hydroxy-4-aminopentanoyl}]pyrrolidine-2-carboxylate Hydrochloride

By the procedure of Example 1, but using proline methyl ester hydrochloride (41.3 mg, 0.25 mM), N-methylmorpholine (25.3 mg, 0.25 mM), 1-hydroxybenzotriazole (33.0 mg, 0.25 mM), dicyclohexylcarbodiimide (51.5 mg, 0.25 mM) and methylene chloride (20 ml) to give the BOC derivative of the title compound in 151 mg yield.

It was deblocked by the procedure of Example 2 to give the title compound (113 mg).

CLAIMS FOR CONTRACTING DESIGNATED STATES:
AT, BE, CH, DE, FR, GB, IT, LI, LU, NL, SE

1. A compound having the formula

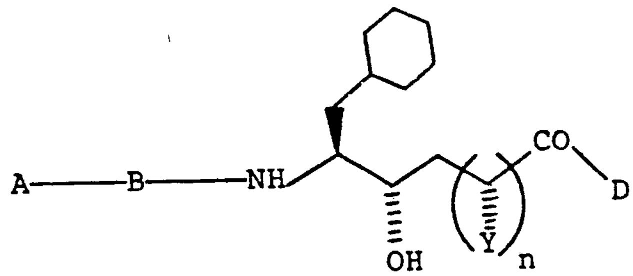

wherein A is

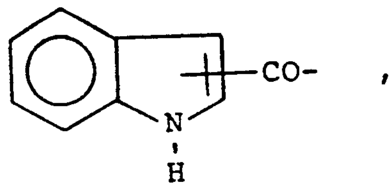

(A-1)

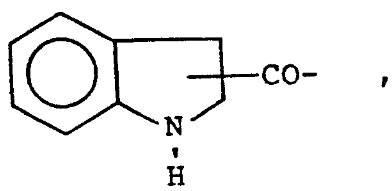

(A-2)

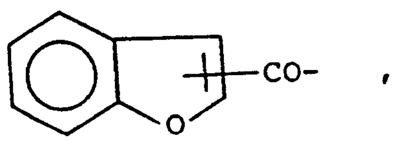

(A-3)

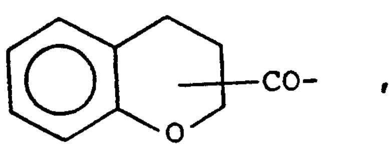

(A-4)

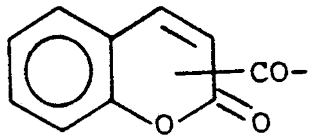 ,

(A-5)

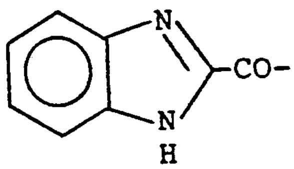 ,

(A-6)

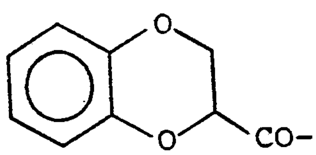 ,

(A-7)

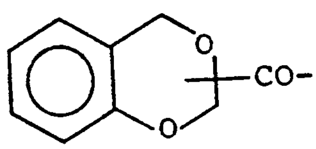 or

(A-8)

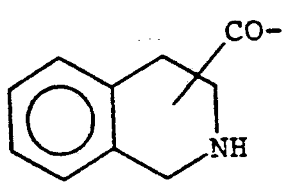 ;

(A-9)

n is 0 or 1;

B is

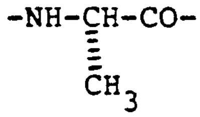 , 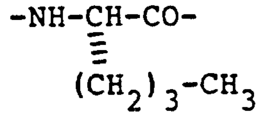 or

(B-1) (B-2)

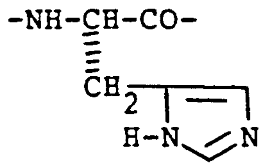 ; (B-3)

D is 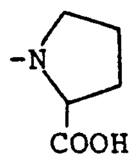 or Z-R

wherein -Z- is -O- or -NH;

R is $(C_{1-6})$alkyl or phenyl each of which can be optionally substituted by amino, hydroxyl, carboxy or carbo$(C_{1-6})$alkoxy provided that when said substituent is amino or hydroxyl, R is $(C_{2-6})$alkyl; and

Y is isobutyl or allyl; or a pharmaceutically acceptable salt thereof.

2. A compound according to claim 1 wherein D is Z-R and is $-NH(C_{1-6})$alkyl optionally substituted by amino, hydroxy or carboxy; B is 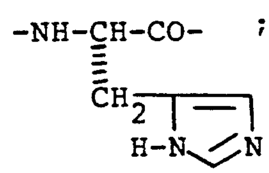 ; n is 1 and A is

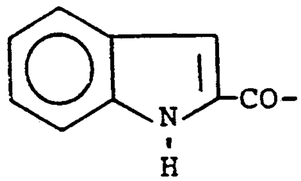

,

(A-1)

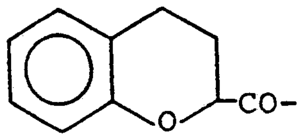

,

(A-4)

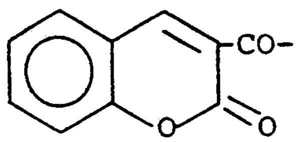

,

(A-5)

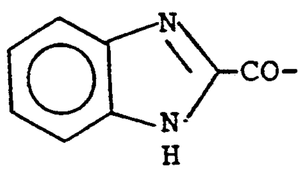

,

(A-6)

or

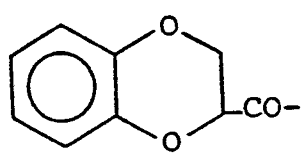

.

(A-7)

3. A compound according to claim 2 wherein D is $-NHCH_3$; Y is isobutyl; and A is (A-1), (A-6) or (A-7).

4. The compound according to claim 2 wherein D is $-NHCH_3$; Y is allyl and A is (A-1), (A-6) or (A-7).

5. A compound according to claim 2 wherein D is $-NH(CH_2)_5NH_2$.

6. The compound according to claim 5 wherein A is (A-6) and Y is isobutyl.

7. A compound according to claim 1 wherein B is $-NH-CH-CO-$; n is 1 and Y is isobutyl.

$CH_3$

8. The compound according to claim 7 wherein D is $-NH(CH_2)_3COOH$ and A is (A-1).

9. A compound according to claim 1 wherein A is (A-2); B is $-NH-CH-CO-$ and n is 0.

$(CH_2)_3-CH_3$

10. The compound according to claim 9 wherein D is $-NH-CH-CH_2OH$.

$CH(CH_3)C_2H_5$

11. A compound having the formula

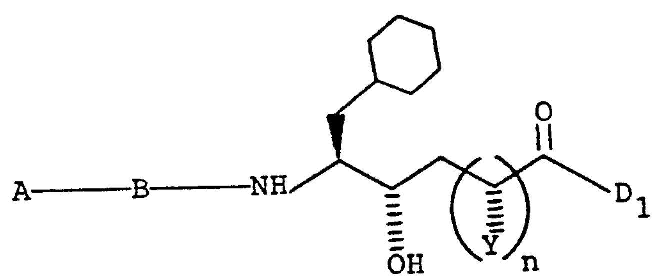

(II)

wherein A is

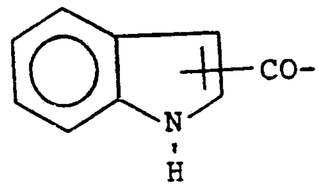 ,

(A-1)

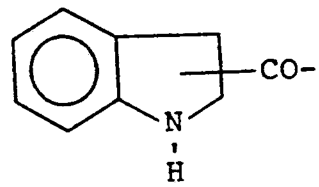 ,

(A-2)

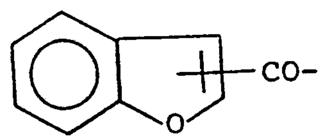 ,

(A-3)

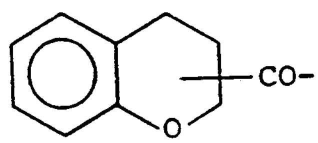 ,

(A-4)

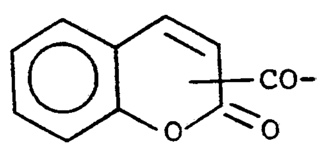 ,

(A-5)

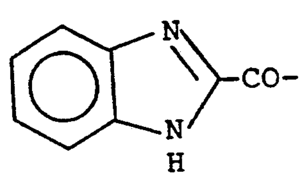 ,

(A-6)

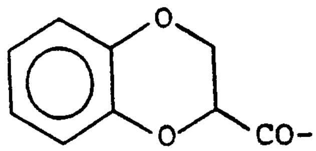 ,

(A-7)

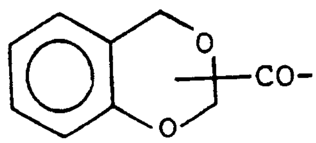 or

(A-8)

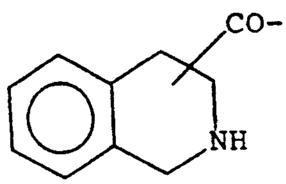 ;

(A-9)

n is 0 or 1;

B is

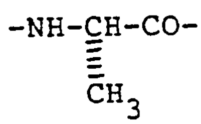 ,

(B-1)

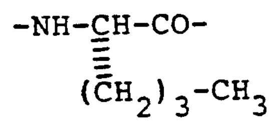 or

(B-2)

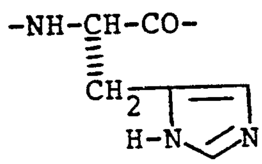 ,

(B-3)

$D_1$ is $(C_{1-6})$alkoxy, OH or benzyloxy;

Y is isobutyl or allyl; or a salt thereof.

12. A compound having the formula

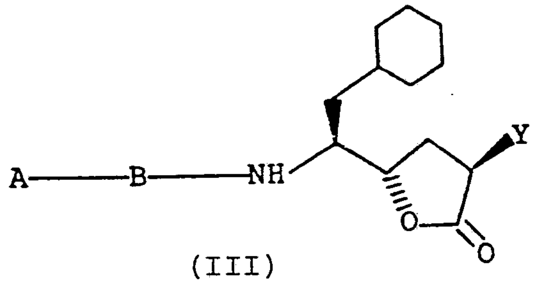

(III)

wherein A is

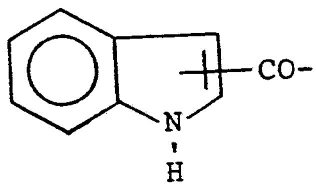

,

(A-1)

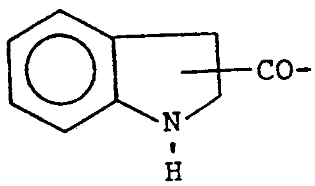

,

(A-2)

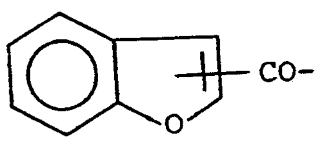

,

(A-3)

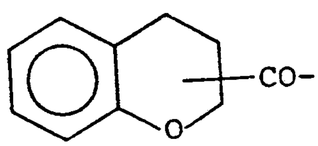

,

(A-4)

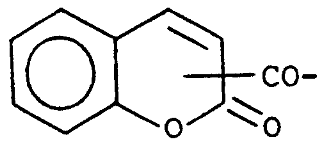 ,

(A-5)

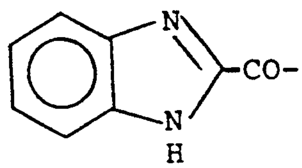 ,

(A-6)

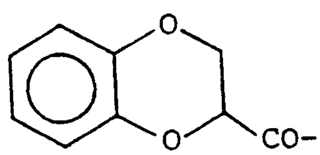 ,

(A-7)

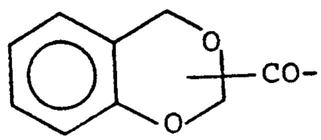 or

(A-8)

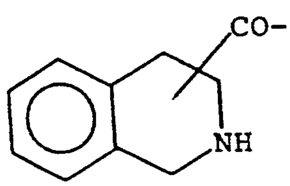 ;

(A-9)

B is

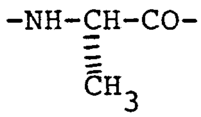

,

(B-1)

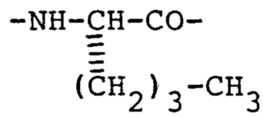

or

(B-2)

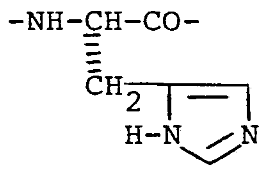

,

(B-3)

Y is isobutyl or allyl; or a salt thereof.

13. A pharmaceutical composition comprising a renin inhibiting-effective amount of a compound of claim 1 in a pharmaceutically acceptable carrier.

14. Compound of formula I as claimed in claim 1 for use as a medicament.

15. Use of a compound of formula I as claimed in claim 1 for the manufacture of a medicament for inhibiting the cleavage of angiotensinogen by renin.

FOR DESIGNATED CONTRACTING STATE: GREECE

1. A process for making a compound having the formula

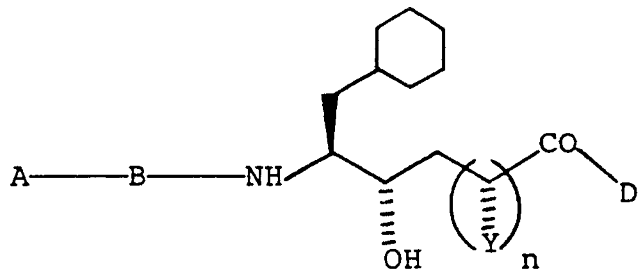

(I)

wherein A is

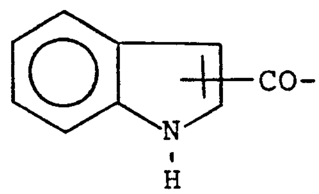

,

(A-1)

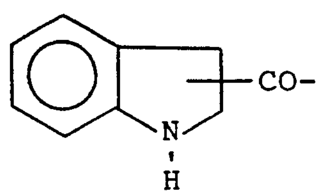

,

(A-2)

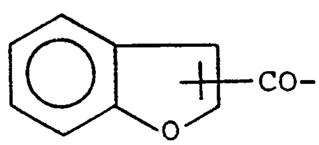

,

(A-3)

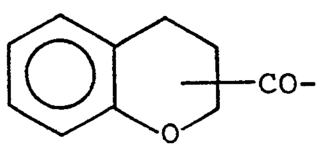

,

(A-4)

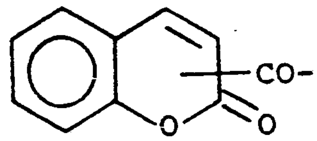 ,

(A-5)

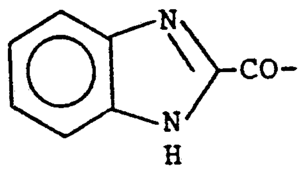 ,

(A-6)

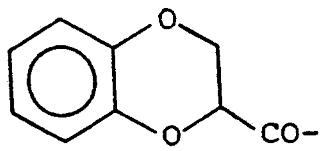 ,

(A-7)

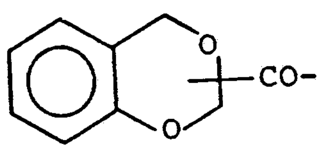 or

(A-8)

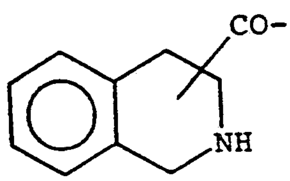 ;

(A-9)

n is 0 or 1;

B is

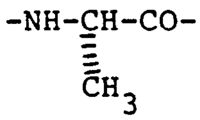 , 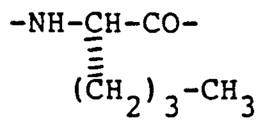 or

(B-1) (B-2)

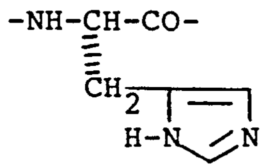 ;

(B-3)

D is 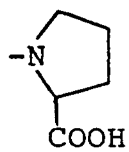 or Z-R

wherein -Z- is -O- or -NH;

R is $(C_{1-6})$alkyl or phenyl each of which can be optionally substituted by amino, hydroxyl, carboxy or carbo$(C_{1-6})$alkoxy provided that when said substituent is amino or hydroxyl, R is $(C_{2-6})$alkyl; and

Y is isobutyl or allyl; or a pharmaceutically acceptable salt thereof which comprises coupling a compound of formula

A-OH

with a compound of formula

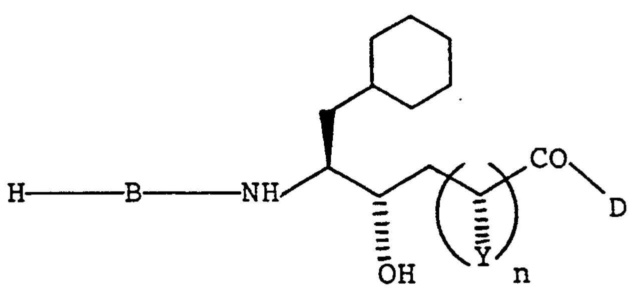

(II)

in the presence of a dehydrative coupling agent in a reaction-inert solvent.

2. A process according to claim 1 wherein A is (A-1), (A-4), (A-5), (A-6) or (A-7); n is 0; D is $-NH(C_{1-6})$alkyl; and B is (B-2) or (B-3).

3. A process according to claim 2 wherein A is (A-1) or (A-6); B is (B-3); Y is isobutyl and D is $-NHCH_3$.

4. A process for making a compound having the formula

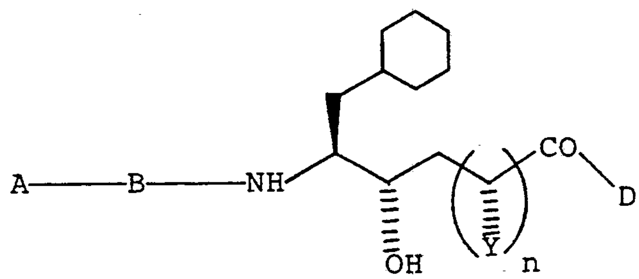

(I)

wherein A is

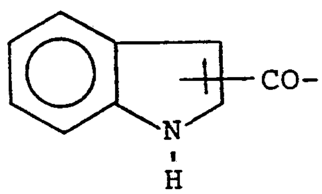

,

(A-1)

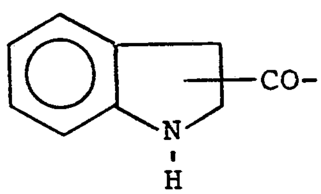

,

(A-2)

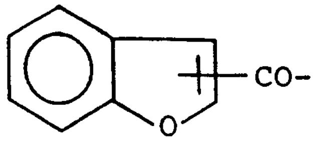 ,

(A-3)

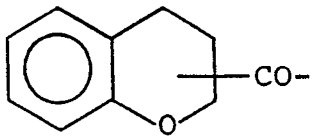 ,

(A-4)

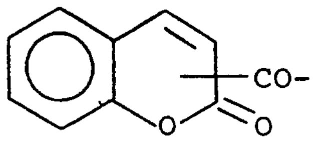 ,

(A-5)

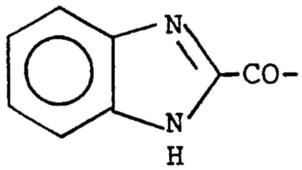 ,

(A-6)

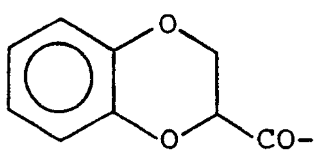 ,

(A-7)

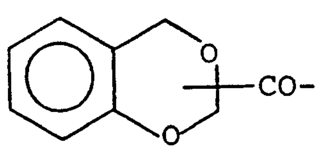 or

(A-8)

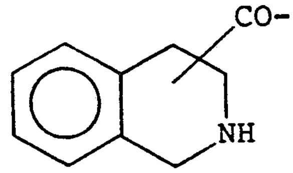 ;

(A-9)

n is 1;

B is

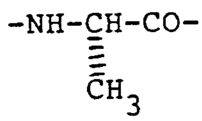 ,

(B-1)

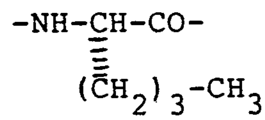 or

(B-2)

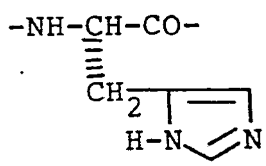 ;

(B-3)

D is

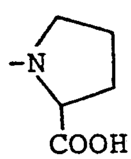 or Z-R

wherein -Z- is -O- or -NH;

R is $(C_{1-6})$alkyl or phenyl each of which can be optionally substituted by amino, hydroxyl, carboxy or carbo$(C_{1-6})$alkoxy provided that when said substituent is amino or hydroxyl, R is $(C_{2-6})$alkyl; and

Y is isobutyl or allyl; or a pharmaceutically acceptable salt thereof which comprises reacting a compound having the formula

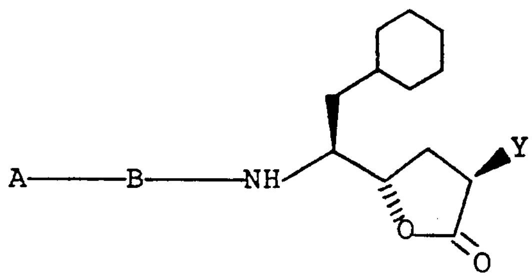

(III)

with a compound having the formula

HD

wherein A, B, Y and D are as defined above in a reaction-inert solvent.

5. A process according to claim 4 wherein A is (A-1), (A-4), (A-5), (A-6) or (A-7); , B is (B-2) or (B-3); and D is $-NH(C_{1-6})$alkyl.

6. A process according to claim 5 wherein A is (A-1) or (A-6); B is (B-3); Y is isobutyl and D is $-NHCH_3$.

7. A compound having the formula

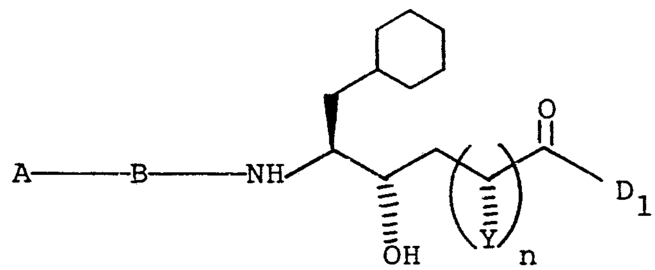

wherein A is

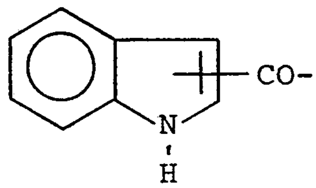 ,

(A-1)

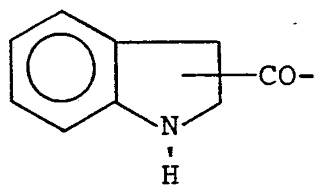 ,

(A-2)

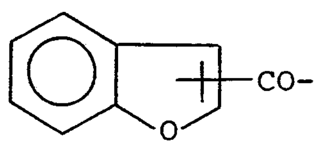 ,

(A-3)

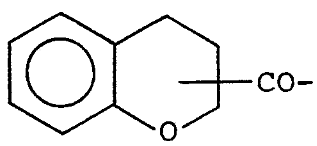 ;

(A-4)

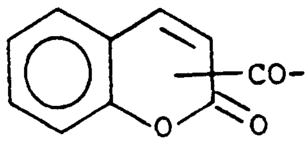

,

(A-5)

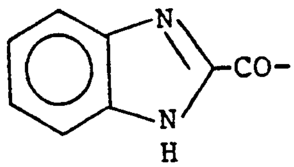

,

(A-6)

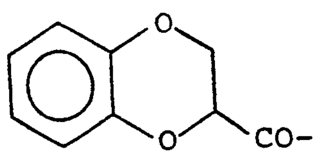

,

(A-7)

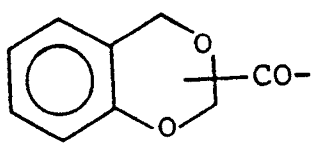

or

(A-8)

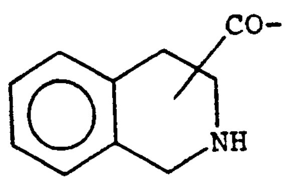

;

(A-9)

n is 0 or 1;

B is

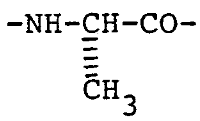 , 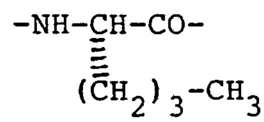 or

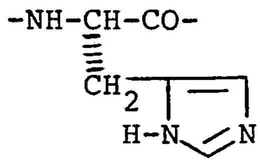 ,

$D_1$ is $(C_{1-6})$alkoxy, OH or benzyloxy;
Y is isobutyl or allyl; or a salt thereof.

8. A compound having the formula

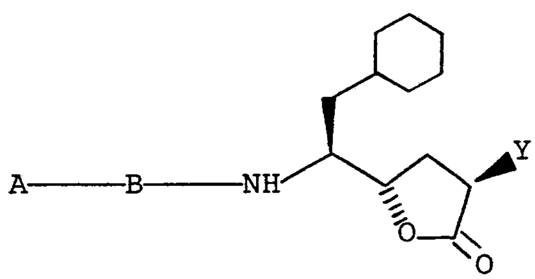

wherein A is

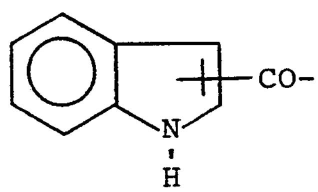 ,

(A-1)

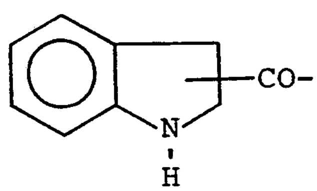 ,

(A-2)

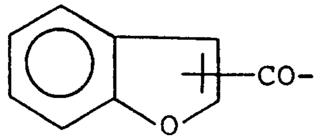 ,

(A-3)

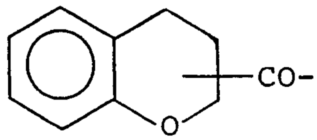 ,

(A-4)

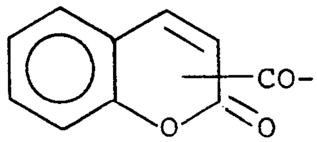 ,

(A-5)

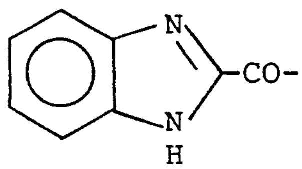 ,

(A-6)

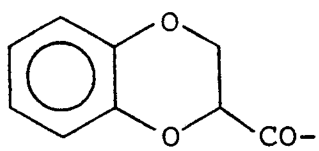 ,

(A-7)

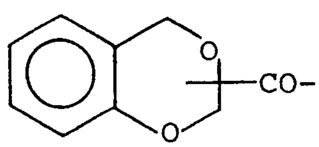 or

(A-8)

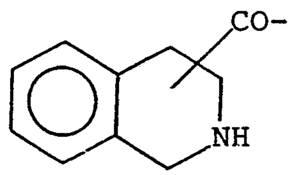 ;

(A-9)

B is

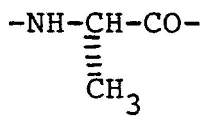 , 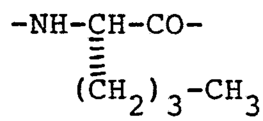 or

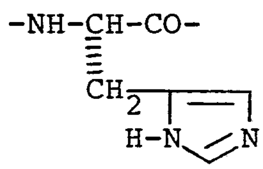 ,

Y is isobutyl or allyl; or a salt thereof.

CLAIMS FOR DESIGNATED CONTRACTING STATE: SPAIN

1. A process for making a compound having the formula

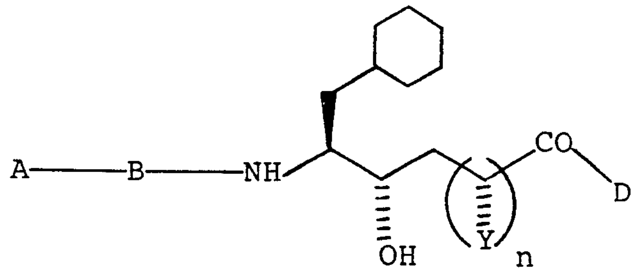

(I)

wherein A is

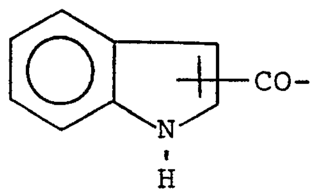

,

(A-1)

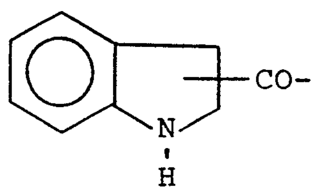

,

(A-2)

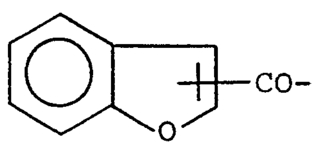

,

(A-3)

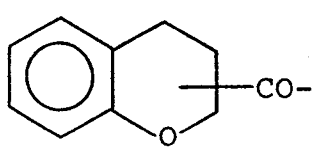

,

(A-4)

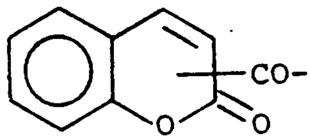 ,

(A-5)

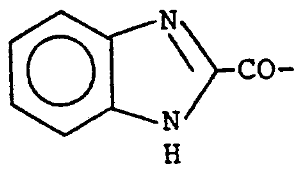 ,

(A-6)

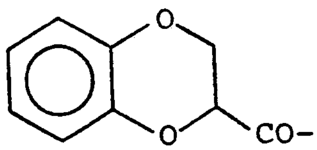 ,

(A-7)

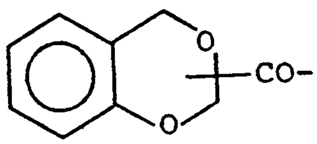 or

(A-8)

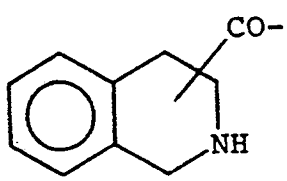 ;

(A-9)

n is 0 or 1;

B is

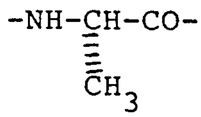

,

(B-1)

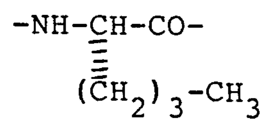

or

(B-2)

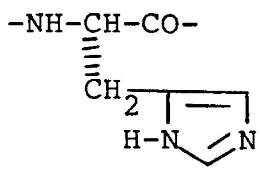

;

(B-3)

D is

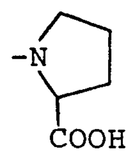

or Z-R

wherein -Z- is -O- or -NH;

R is $(C_{1-6})$alkyl or phenyl each of which can be optionally substituted by amino, hydroxyl, carboxy or carbo$(C_{1-6})$alkoxy provided that when said substituent is amino or hydroxyl, R is $(C_{2-6})$alkyl; and

Y is isobutyl or allyl; or a pharmaceutically acceptable salt thereof which comprises coupling a compound of formula

A-OH

with a compound of formula

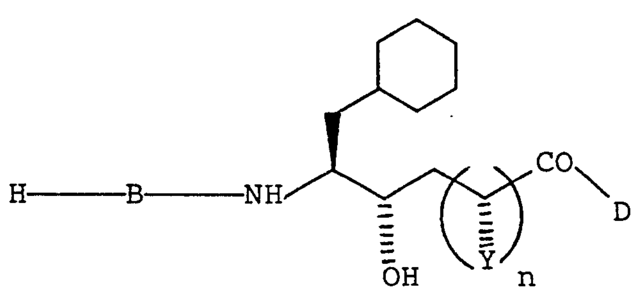

in the presence of a dehydrative coupling agent in a reaction-inert solvent.

2. A process according to claim 1 wherein A is (A-1), (A-4), (A-5), (A-6) or (A-7); n is 0; D is $-NH(C_{1-6})$alkyl; and B is (B-2) or (B-3).

3. A process according to claim 2 wherein A is (A-1) or (A-6); B is (B-3); Y is isobutyl and D is $-NHCH_3$.

4. A process for making a compound having the formula

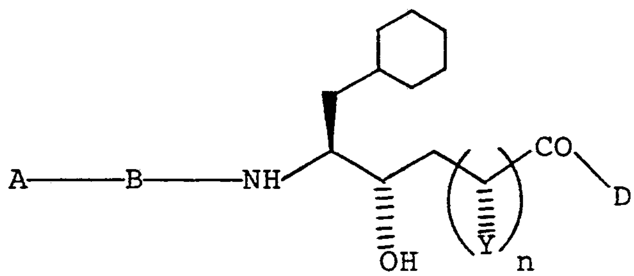

(I)

wherein A is

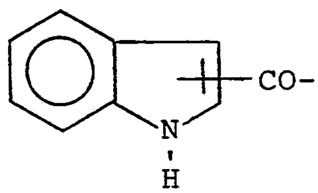

,

(A-1)

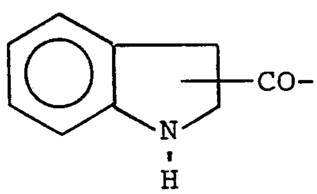

,

(A-2)

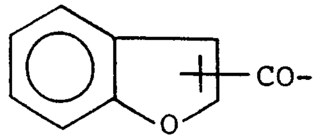

,

(A-3)

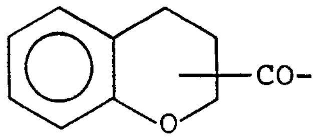

,

(A-4)

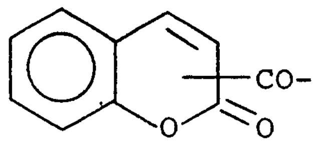

,

(A-5)

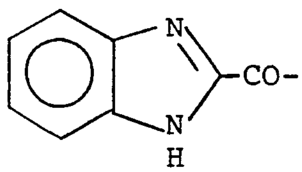

,

(A-6)

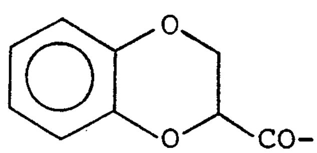

,

(A-7)

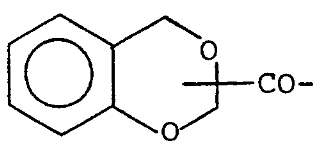

or

(A-8)

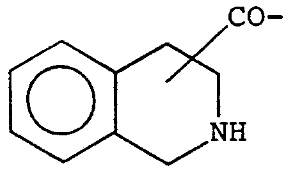 ;

(A-9)

n is 1;

B is

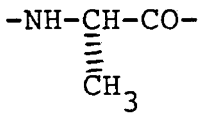 , 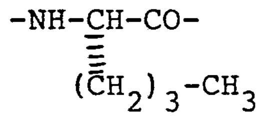 or

(B-1) (B-2)

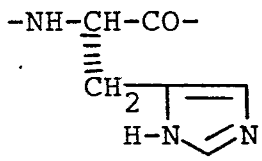 ;

(B-3)

D is 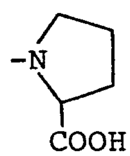 or Z-R

wherein -Z- is -O- or -NH;

R is $(C_{1-6})$alkyl or phenyl each of which can be optionally substituted by amino, hydroxyl, carboxy or carbo$(C_{1-6})$alkoxy provided that when said substituent is amino or hydroxyl, R is $(C_{2-6})$alkyl; and

Y is isobutyl or allyl; or a pharmaceutically acceptable salt thereof which comprises reacting a compound having the formula

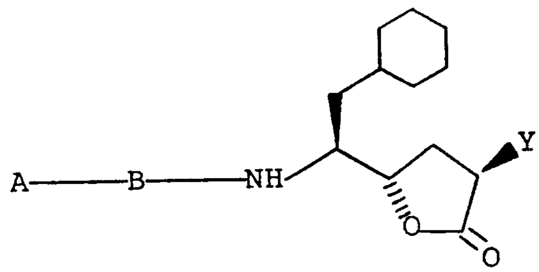

(III)

with a compound having the formula

HD

wherein A, B, Y and D are as defined above in a reaction-inert solvent.

5. A process according to claim 4 wherein A is (A-1), (A-4), (A-5), (A-6) or (A-7); B is (B-2) or (B-3); and D is $-NH(C_{1-6})$alkyl.

6. A process according to claim 5 wherein A is (A-1) or (A-6); B is (B-3); Y is isobutyl and D is $-NHCH_3$.

7. A process for preparing compounds of formula (III)

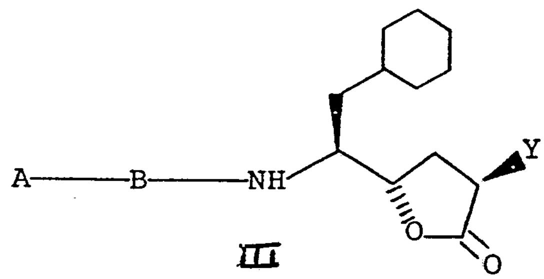

III

wherein A is

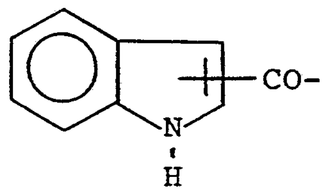

,

(A-1)

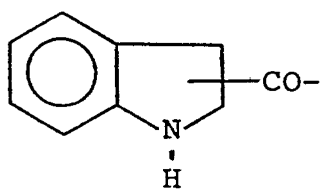

,

(A-2)

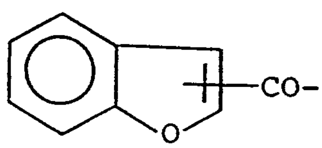

,

(A-3)

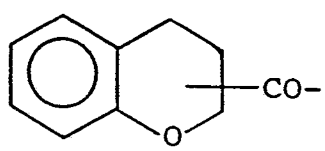

,

(A-4)

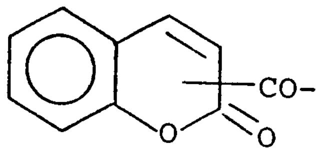
,

(A-5)

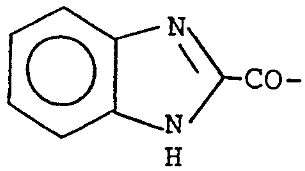
,

(A-6)

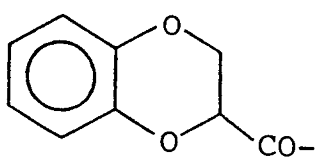
,

(A-7)

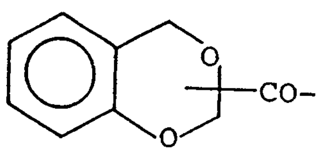
or

(A-8)

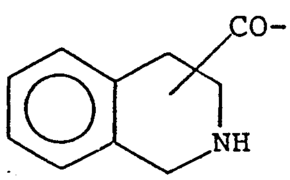
;

(A-9)

B is

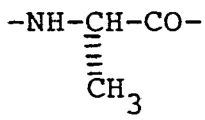 , 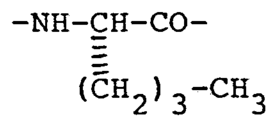 or

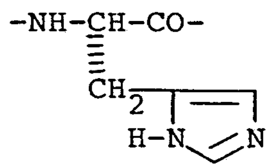 ,

Y is isobutyl or allyl; or a salt thereof.
by reacting a compound of formula

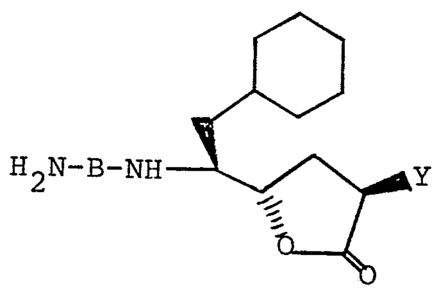

with a compound of formula

A-COOH

in a reaction inert solvent wherein A, B, Y and D are as defined above.